# EUROPÄISCHE PATENTSCHRIFT

(11) **EP 3 762 705 B1**
(45) Veröffentlichungstag und Bekanntmachung des Hinweises auf die Patenterteilung: **02.09.2026**
(21) Anmeldenummer: 19709923.7
(22) Anmeldetag: 08.03.2019
(51) Int. Cl.: G01N 15/08, G01N 33/24, G01V 9/00, G01V 9/02, G01K 11/06

(54) **VERFAHREN ZUR QUANTIFIZIERUNG VON PORÖSEN MEDIEN MITTELS ANALYSEPARTIKEL UND DEREN ANWENDUNGEN**
METHOD FOR QUANTIFYING POROUS MEDIA BY MEANS OF ANALYTICAL PARTICLES AND USES THEREOF
PROCÉDÉ SERVANT À QUANTIFIER DES MILIEUX POREUX AU MOYEN DE PARTICULES D'ANALYSE ET DE LEURS APPLICATIONS

(30) Priorität: 08.03.2018 DE 102018105394
(43) Veröffentlichungstag der Anmeldung: 13.01.2021
(73) Patentinhaber: Patentpool Innovations Management GmbH, 80331 München (DE)
(72) Erfinder: SCHIMMEL, Thomas, 76131 Karlsruhe (DE)
(74) Vertreter: Müller-Boré & Partner Patentanwälte PartG mbB
(86) Internationale Anmeldenummer: PCT/EP2019/055842
(87) Internationale Veröffentlichungsnummer: WO 2019/170856

(56) Entgegenhaltungen:
- EP-A2- 1 862 791
- WO-A1-2016/007170
- RU-C1- 2 504 403
- TW-A- 201 513 953
- TW-A- 201 513 953
- US-A1- 2014 000 357
- US-A1- 2015 001 385

## Beschreibung

Die Erfindung betrifft ein Verfahren zur Quantifizierung von porösen Medien mit speziell hierfür konzipierten Analysepartikel (nachfolgend auch "Partikel" genannt) und deren Anwendungen, beispielsweise zur Bestimmung der Wasserdurchlässigkeit von Gesteinen als Voraussetzung für die Entwicklung von Kriterien zur Grundwasserbewegung oder der stofflichen Charakterisierung von porösen Materialien oder Gesteinsschichten oder zur Überwachung chemischer, biologischer und/oder biotechnologischer Reaktoren, oder in medizinischen In-vivo-Verfahren.

Aus dem Stand der Technik sind Tracersysteme in der Hydrologie bekannt. Grundlage der Hydrogeologie, zur Begriffsbestimmung gemäß DIN 4049-1 und/ oder DIN4039-3, ist die Kenntnis des Aufbaus des Untergrundes. Basis dafür sind geologische Karten, Ergebnisse von Bohrungen und dreidimensionale Modelle des geologischen Untergrundes. Werkzeuge und Methoden zur Erkundung und Überwachung (Monitoring) des Grundwassers sind Fernerkundung (remote sensing), geophysikalische Methoden, Bohrungen, Grundwassermessstellen, Pumpversuche, Laborversuche und die wasserchemische Analytik. Grundwasser in klüftigem Gestein oder porösen Materialien kann laminar, aber auch turbulent fließen.

Da die Fließwege und ihre Eigenschaften in Gebirgen oder porösen Materialien selten gänzlich bekannt sind, ist eine Abschätzung ihrer Anteile an der gesamten Grundwasserströmung schwierig. Insgesamt wird aber der Einfluss von turbulenter Strömung auf die hydraulische Durchlässigkeit und die Strömungsgeschwindigkeit als gering angesehen. Deswegen werden Kluftgrundwasserleiter vereinfachend meist mit dem Kontinuum-Modell und die Bewegung als ausschließlich laminar beschrieben. Bei den hydraulischen Methoden wird mit einem bekannten Signal (positive oder negative Druckänderung) auf ein meist unbekanntes System, den Grundwasserleiter oder auch Aquifer genannt, eingewirkt, zum Beispiel mit einem Langzeitpumpversuch.

Die Reaktion des Systems (Druckabsenkung, Druckanstieg bzw., Wasserspiegelabsenkung, - anstieg) wird während des Versuchs pro Zeiteinheit aufgezeichnet. Nach Signalende kann auch die Rückkehr des Systems in seinen Gleichgewichtszustand gemessen werden. Bei Langzeitpumpversuchen oder Injektionsversuchen werden die dem Aquifer mitgeteilte Wasserstands- oder Druckänderung über einen längeren Zeitraum beobachtet. Nach STOBER (1993) können nur bei Langzeitpumpversuchen Aussagen über das Strömungsverhalten bzw. über das Aquifermodell gemacht werden. Bei allen anderen hydraulischen Tests wird das Modell der Auswertung implizit vorgegeben.

Packertest von Packer (Bohrung), Sammelbegriff für hydraulische Versuche im Bohrloch oder Brunnen, die beim Einfachpackertest im oberhalb bzw. unterhalb des Packers befindlichen und hydraulisch abgetrennten Abschnitt, beim Doppelpackertest auch abschnittsweise in der Strecke zwischen zwei Packern durchgeführt werden. Einfachpackertests werden in teiloffenen Bohrlöchern durchgeführt, die bis unter den Wasserspiegel verrohrt sind. Durch den Packer führt eine dünnere Rohrtour, über die für den unteren Bohrlochabschnitt gültige Auffüllversuche, Slug-Tests oder Einschwingverfahren zur Ermittlung der hydraulischen Kennwerte des Grundwasserleiters gefahren werden können. Doppelpackertests werden im unverrohrten Bohrloch durchgeführt, wobei der Streckenabschnitt zwischen den beiden Packern mit den oben angeführten Versuchen getestet werden kann.

Die Zuverlässigkeit der Ergebnisse hängt dabei von der Dichtigkeit der Packer, der Beschaffenheit der Bohrlochwand und des umgebenden Gebirges ab bzw. kann durch Unterläufigkeit und /oder auch Umläufigkeit im Gebirge herabgesetzt sein. Zu felsmechanische Untersuchungen werden Wasserdruck-Tests über einfach oder doppelt abgepackerte Bohrlochabschnitte eingesetzt, bei denen Wasser unter unterschiedlichen Drücken zur Ermittlung des kf-Wertes von Festgesteinen, z.B. im Untergrund von Staudämmen, eingepresst wird *(Lexikon der Geowissenschaften, Spektrum Verlag*)*.* Die Permeabilität K wird in der Geotechnik zur Quantifizierung der Durchlässigkeit von Böden und Fels für Flüssigkeiten oder Gase (z. B. Grundwasser, Erdöl oder Erdgas) benutzt. Mit ihr sehr eng verbunden ist der hier gleichzeitig erläuterte Durchlässigkeitsbeiwert kf-Wert. Auch der Durchlässigkeitsbeiwert (bzw. die hydraulische Leitfähigkeit) quantifiziert die Durchlässigkeit von Boden oder Fels, jedoch gehen hier zusätzlich die Dichte und die Viskosität des durchströmenden Fluids ein (Bernward Hölting, Wilhelm G. Coldewey: Hydrogeologie: Einführung in die Allgemeine und Angewandte Hydrogeologie. 6. Auflage. Elsevier Spektrum Akademischer Verlag, München 2005*).*

Auch werden in der Hydrologie Tracer-Verfahren eingesetzt mit oder ohne Kombination von Pumpversuchen. Unter einem Tracer (engl. trace = Spur) wird eine künstliche oder natürliche Substanz (Tracer-Stoff) verstanden, die nach Einbringung in ein hydrologisches System unterschiedlichste Untersuchungen ermöglicht oder erleichtert.

Elektrolytischer Tracer (NaCl, MgCl₂): Der Tracer-Stoff wird in einen oberstromigen Pegel (Messstelle) oder Schwinde (Versickerungsstrecke) eingegeben. Im Brunnen werden Widerstandsmessungen durchgeführt. Dabei wird die Zunahme der Ionenkonzentration von Grundwasserzutritten in der Zeit gemessen. Möglich ist auch die Messung der Verdünnung im Eingabepegel.

Sporendrift, Mikropartikel: Lycopodiumsporen (Bärlappsporen) werden mit bis zu fünf verschiedenen Farben eingefärbt. Damit können verschiedene Schwinden mit unterschiedlich gefärbten Sporen gleichzeitig als Eingabestellen verwendet werden. Durch ihre Dichte von 1,1 bleiben die Sporen lange in der Schwebe. Ihre Größe beträgt ca. 33 Mikrometer [µm]. Das Auffangen der Sporen erfolgt mit einem Planktonnetz, das in der Regel täglich gelehrt werden muss. Die Auswertung wird mikroskopisch durch Auszählen vorgenommen. Mikropartikel wie Polystyrol-Kügelchen stehen in Größen von 0,05 bis 90 Mikrometer [µm] zur Verfügung. Sie sind mit Fluoreszenzfarben eingefärbt und können wie eingefärbte Sporen eingesetzt werden. Untersuchungsziel: Die Vorteile des Verfahrens sind, dass es weder die Wasserqualität noch das Aussehen des Wassers beeinträchtigt, und die Möglichkeit aufgrund der Sporenfarben verschiedene Schwinden auseinanderhalten zu können.

Die Nachteile sind, dass nur qualitative Aussagen über die Fließwege und Fließgeschwindigkeiten getroffen werden können. Die Sporen- bzw. Partikelgröße begrenzt die Drift auf offene Klüfte mit einer Mindestöffnungsweite und Karsthohlräumen. Poröse Sedimente, Gesteine oder Materialien mit kleinerer Porengröße halten die Sporen oder Polystyrol-Kügelchen zurück.

Aktivierungsanalytisches Tracer-Verfahren: Für die Eingabe in die Schwinde wird dem versickernden Wasser eine nicht radioaktive Substanz zugemischt und erst die gezogenen Wasserproben zur Aktivierung des Elements im Reaktor unter Neutronenbeschuss gesetzt. Als Tracer-Stoffe kommen Brom als Ammoniumbromid (NH₄Br), Lanthan oder Indium in Frage. Bei Indium kann mit einer Tracer-Menge im Bereich von mehreren 100 g gerechnet werden (ZÖTL, 1974). Die Vorteile dieses Verfahrens liegen in der Verwendung von ungiftigen nicht radioaktiven Stoffen, die nur in relativ kleinen Mengen eingesetzt werden müssen. Dadurch sind während der Versuchsdurchführung keine besonderen Sicherheitsmaßnahmen erforderlich. Die Auswertung erfolgt wie bei den anderen Tracer-Verfahren durch ein Konzentrations- / Zeit-Diagramm. Damit kann zwischen dem Ort der Tracer-Eingabe und dem Ort der Tracer-Messung die Abstandsgeschwindigkeit des Grundwassers bestimmt werden.

Einbohrloch-Verdünnungs-Methode mit radioaktiven Tracern: In einem durch Packer abgesperrten Bohrlochbereich wird die Konzentrationsabnahme eines zuvor zugefügten Tracers gemessen. Aus der zeitlichen Konzentrationsabnahme im Packerintervall kann eine Verdünnungsgeschwindigkeit abgeleitet werden. Die Verdünnungsgeschwindigkeit steht in einem empirischen Zusammenhang mit der herrschenden Filtergeschwindigkeit. Entsprechend des Bohrlochausbaues (Filterrohrtyp, Schlitzweite usw.) müssen Korrekturen der Messergebnisse durchgeführt werden. Wird als Tracer radioaktives Material verwendet, kann die horizontale Grundwasserfließrichtung ermittelt werden, indem nach dem Abströmen des Tracers in den Grundwasserleiter die radioaktive Strahlung durch eine winkelabhängige Detektion ermittelt werden kann. Radioaktive Tracer sind aber bei Trinkwasserfassungen problematisch.

Einbohrloch-Verdünnungs-Methode mit Uranin als Tracer Messverfahren: Das Institut für Wasserbau der Universität Stuttgart (MARSCHALL 1993) hat ein Lichtleiterfluorometer zur In-situ-Konzentrationsmessung entwickelt. Dadurch können auch Fluoreszenzfarbstoffe wie Uranin eingesetzt werden. Die Nachweisgrenze bei diesen Stoffen liegt sehr viel niedriger (10⁻⁵-10⁻⁶ mg/l) als bei Leitfähigkeitsmessungen mit NaCl-Tracern (0,1 - 1 mg/I). Damit kann noch eine horizontale Filtergeschwindigkeit von 10⁻⁷ m/s gemessen werden.

Auch der Einsatz eines Farbstoffes als Tracer ist beschrieben. Der Tracer-Stoff wird als Farbstoff in einen oberstromigen Pegel (Messstelle) oder Schwinde (Versickerungsstrecke) zur Untersuchung von Strömungen und Fließrichtung eingegeben. Die Donauversickerung wurde bereits im Jahre 1877 mit Fluorescein als Tracer-Molekül aufgeklärt.

Die Grundwassergeschwindigkeit wird maßgeblich durch die miteinander verbundenen Gesteinseigenschaften, wie Gebirgsdurchlässigkeit (Wasserdurchlässigkeit im natürlichen Gesteinsverband, Gesteinsspalten und durchflusswirksames Hohlraumvolumen) beeinflusst. Daten zu diesen Größen stehen aber zu Beginn der Standortauswahl weder flächendeckend zur Verfügung noch können sie für alle zu betrachtenden Standorte mit ausreichender Belegdichte erhoben werden.

Nachteile sind die geringe horizontale Eindringtiefe (Reichweite) in die Gebirgsformation oder poröse Materialien. Der Speicherkoeffizient kann nur abgeschätzt werden. Aussagen über Aquifer-Modell, Aquifer-Ränder und Anisotropie sind nur begrenzt mit speziellen Auswerteverfahren möglich. Die Genauigkeit der Messergebnisse hängt in erster Linie von der Qualität der Datenerfassung ab, nicht aber von den eingesetzten Tracern an sich. Besonders bei geringer Durchlässigkeit sind genaue Druck- und Mengenmessungen entscheidend.

Solche Messungen in Gesteinsritzen, Felsen, geologischen Formationen, porösen Materialien oder bei Bohrungen, um vor Ort Parameter wie Druck, Temperatur zu messen, sind auf Grund der Unzugänglichkeit nur schwer oder gar nicht möglich und gegebenenfalls nur für sehr kleine Messsonden geeignet. Miniaturisierte elektrische oder optische Sensoren und Messgeräte, die an solchen Orten und unter solchen Bedingungen messen können, lassen sich nur sehr schwer und mit hohem Aufwand herstellen und in das Gestein einbringen und nur schwer mit einer externen Messelektronik verbinden.

Eine konventionelle Bestimmung mit Messsonden, Sensoren, Detektions-Elektronik scheidet meist aus, da diese zum einen mit Energie versorgt werden müssen und zum anderen die Daten ausgelesen werden müssen. Beides würde eine Versorgung mit Strom- und Signalleitungen erfordern, die eigene Bohrungen erforderlich machen, was nicht nur einen enormen technischen und finanziellen Aufwand erfordern würde, sondern auch die Messgrößen und die Strömungsbedingungen (beispielsweise von Wasser in diesen Gesteinsschichten) verfälschen würde. Der Einsatz von Tracer aus dem Stand der Technik führt nur zu einer passiven Messung von Parametern, wie Fließ- oder Strömungsgeschwindigkeit, Verdünnung oder Durchlässigkeit.

Eine Quantifizierung von Parameter wie Druck, Temperatur oder anderer physikalischer und/oder chemischer Parameter beim Durchgang durch das Gestein und/oder Gesteinsschichten oder porösen Materialien, beziehungsweise welchen physikalischen, chemischen und biochemischen Bedingungen diese auf seinem Weg ausgesetzt waren, werden nicht erfasst.

US 2014/000357 A1 offenbart ein Verfahren zum Schätzen der Eigenschaften einer unterirdischen Formation, die von einem Bohrloch durchdrungen wird, welches die folgenden Schritte umfasst: Einführen eines Injektionsfluids in das Bohrloch und die unterirdische Formation, wobei das Injektionsfluid mehrere Tracer-Mittel umfasst, wobei jedes Tracer-Mittel ein Objekt im Submikron-Maßstab ist, und das Bestimmen der Eigenschaften der Formation durch Analysieren von Änderungen der Verteilungsfunktionen in der Konzentration, Größe und Art der Tracer-Mittel nachdem das Injektionsfluid aus der unterirdischen Formation zurückgewonnen wurde. EP 1 862 791 A2 offenbart ein Verfahren zur Kontrolle oder zur Bestimmung der Charakteristika einer Mikrofiltrations- oder Ultrafiltrationsmembran, dadurch gekennzeichnet, dass man durch die Membran wenigstens eine Menge einer stabilisierten wässrigen Suspension aus mineralischen magnetischen Nanopartikeln, umfassend ein Stabilisierungsmittel, mit gegebener Konzentration passieren lässt, deren Partikelabmessung 1 bis 100 nm mit einem bestimmten d50 Wert beträgt. TW 201 513 953 A offenbart ein Herstellungsverfahren für eine Wasserlösung, die Metall-Nanopartikel enthält. RU 2 504 403 C1 offenbart ein Herstellungsverfahren für eine Wassersuspension von nanokristallinen Siliziumpartikeln für biomedizinische Anwendungen. WO 2016/007170 A1 offenbart Verfahren zum Analysieren einer porösen Gesteinsprobe, wobei das Verfahren das Injizieren einer Suspension von Nanopartikeln in eine Gesteinsprobe, wobei die Gesteinsprobe einen in einem Gesteinsmedium definierten Porenraum umfasst; das Erhalten eines Bildes der Nanopartikel im Porenraum der Gesteinsprobe; und die Analyse des Porenraums der Gesteinsprobe anhand des Bildes umfasst. US 2015/001385 A1 offenbart ein Verfahren zum Untersuchen eines geologischen Untergrundbereichs durch Diffusion einer Injektionsflüssigkeit in den Untergrundbereich, wobei das Verfahren umfasst: Injizieren einer Injektionsflüssigkeit in den zu untersuchenden unterirdischen Bereich, wobei die Injektionsflüssigkeit Nanopartikel umfasst, die in der Lage sind, eine stabile kolloidale Suspension in einem salzhaltigen Medium zu bilden, wobei die Nanopartikel einen mittleren Durchmesser zwischen 20 und 200 nm aufweisen und aus Folgendem bestehen: einen Kern und gegebenenfalls eine Matrix, die den Kern umhüllt; und mindestens eine fluoreszierende Einheit, die in der Lage ist, ein oder mehrere Fluoreszenzsignale mit Memory-Effekt zu erzeugen, so dass das Fluoreszenzsignal aufgrund der im unterirdischen Bereich herrschenden physikochemischen Bedingungen irreversibel verändert werden kann; Auffangen der diffundierten Injektionsflüssigkeit zu verschiedenen Zeitpunkten nach einer Injektionsperiode; und das Erfassen und Analysieren der von den Nanopartikeln emittierten Fluoreszenzsignale mit Memory-Effekt, wobei die Analyse des Memory-Effekts der erfassten Fluoreszenzsignale es ermöglicht, daraus Informationen über die physikochemischen Bedingungen des untersuchten unterirdischen geologischen Bereichs abzuleiten.

Davon ausgehend liegt die Aufgabe der Erfindung darin ein neuartiges Verfahren zur Quantifizierung eines porösen Mediums bereitzustellen, wodurch die vorstehend genannten Nachteile der konventionellen Methoden vermieden werden können.

Die Aufgabe wurde durch ein Verfahren zur Quantifizierung eines porösen Mediums mit mindestens einem Partikel oder einer Mischung aus Partikeln, wobei die Partikel eine Referenzfunktion und mindestens eine Berichtsfunktion zum Erfassen von physikalischen, chemischen oder biochemischen Parametern des porösen Mediums aufweisen, gelöst, welches Verfahren die folgenden Schritte umfasst:
das Einbringen des Partikels und/oder der Partikelmischung in ein Fluid,
das Durchströmen und/oder die Permeation des Fluids mit dem Partikel und/oder der Partikelmischung durch das poröse Medium, wobei sich die mindestens eine Berichtsfunktion der Partikel bei Überschreiten oder Unterschreiten eines Schwellwertes des zu erfassenden Parameters verändert, während die Referenzfunktion der Partikel unverändert bleibt, und
nach Austritt aus dem porösen Medium, mindestens eine nachfolgende Analyse des Partikels und/oder der Partikelmischung auf die physikalisch, chemisch oder biochemisch veränderte Berichtsfunktion und der Referenzfunktion der Partikel, wobei die Referenzfunktion zur Wiedererkennung der Partikel dient,
wobei die Referenzfunktion der Partikel in Form eines Markers für die plasmonische Eigenschaft vorliegt,
wobei die mindestens eine Berichtsfunktion in den Partikeln und/oder auf der Partikeloberfläche enthalten ist, umfassend mindestens einen Fluoreszenz-Marker oder einen Marker für die plasmonische Eigenschaft,
wobei der Fluoreszenz-Marker Fluoreszenzfarbstoffe, fluoreszierende Moleküle, fluoreszierende Molekülgruppen oder Quantum Dots einschließt,
wobei der Marker für die plasmonische Eigenschaft metallische Nanopartikel, die plasmonische Resonanzen zeigen, einschließt, und
wobei die Veränderung der mindestens einen Berichtsfunktion irreversibel ist.

Das erfindungsgemäße Partikel ist auf der Sub-Millimeter-Skala, der Mikrometer-Skala oder der Nanometerskala und umfasst mindestens eine Referenzfunktion und mindestens eine Berichtsfunktion zum Erfassen von physikalischen, chemischen oder biochemischen Parametern des porösen Mediums.

Vorzugsweise weisen die Partikel einen Durchmesser von 100 µm bis 0,5 nm auf, besonders bevorzugt einen Durchmesser von 10 µm bis 5 nm, oder ganz besonders bevorzugt einen Durchmesser von 5 µm bis 50 nm.

Vorzugsweise besteht der Grundkörper der Partikel aus Silber, Gold, Kupfer oder anderen Metallen, Siliziumoxid, Polystyrol, Olefinen, Wachs oder einer Mischung daraus.

Gemäß der vorliegenden Erfindung umfasst die Berichtsfunktion mindestens einen Fluoreszenz-Marker oder einen Marker für die plasmonische Eigenschaft.

Vorzugsweise steigt die Veränderung der mindestens einen Berichtsfunktion kontinuierlich mit der erfahrenen Dosis der Strahlung (Strahlenbelastung) oder der oxidativen Belastung an.

Vorzugsweise weisen die Partikel ferner eine Zusatzfunktion auf, die anhand eines zeitabhängigen Zerfalls oder einer zeitabhängigen Veränderung einer Eigenschaft die Verweilzeit des Partikels in dem porösen Medium zu bestimmen erlaubt.

Vorzugsweise weisen die Partikel ferner eine magnetische Zusatzfunktion auf.

Vorzugsweise weisen die Partikel eine weitere Berichtsfunktion auf, die sich bei Überschreiten oder Unterschreiten eines Schwellwertes eines zweiten zu erfassenden Parameters, der von dem ersten Parameter verschieden ist, verändert.

Vorzugsweise ist das poröse Medium ein flüssigkeits- oder gasgefüllter Raum.

Weiterhin ist es bevorzugt, dass das poröse Medium Gesteine, Gesteinsschichten und/oder ein poröses Material oder Schichten aus diesem porösen Material umfasst.

Die Analyse des Partikels und/oder der Partikelmischung erfolgt vorzugsweise durch optische Spektroskopie, IR-Spektroskopie, plasmonischer Resonanz, Mikroskopie, Dosimetrie, Kernspinresonanz, Elektronenspinresonanz, ENDOR, Fluoreszenzspektroskopie, Einzelmolekülfluoreszenzspektroskopie, Atom-Fluoreszenzspektroskopie, Lumineszenz-Spektroskopie, Photolumineszenz-Spektroskopie, Chromatographie, Gaschromatographie, Flüssigkeitschromatographie und/oder Hochleistungsflüssigkeitschromatographie (HPLC) erfolgt, oder anhand einer Folgereaktion, die den Nachweis der Veränderung der Berichtsfunktion erleichtert.

Die vorliegende Erfindung betrifft weiterhin die Verwendung des erfindungsgemäßen Verfahrens zur Quantifizierung von Gesteinen, Gesteinsschichten und/oder porösen Materialien oder Schichten aus diesem porösen Material, bei der geologischen Untersuchung von Gesteinen, Gesteinsschichten und/oder porösen Materialien oder Schichten aus diesem porösen Material, in der Hydrologie, Gewässererkundung, Lagerstättenerkundung, Lagerstättenüberwachung, Fracking, Geothermie, Dichtigkeitsüberwachung, Überwachung chemischer, biologischer und/oder biotechnologischer Reaktoren, von Wasserbehältern, Wasserspeichern und Wasserleitungssystemen oder in medizinischen In-vivo-Verfahren.

Wie vorstehend beschrieben weist das Partikel eine Referenzfunktion und mindestens eine Berichtsfunktion zum Erfassen von physikalischen, chemischen oder biochemischen Parametern auf. Die Referenzfunktion stellt eine durch die physikalischen, chemischen oder biochemischen Parameter unveränderte Reporting-Funktion dar und dient der Wiedererkennung des bzw. der Partikel(s) nach dessen bzw. deren Austritt aus dem porösen Medium.

Die Berichtsfunktion ändert sich sobald die Bedingungen, die das Partikel bei seinem Durchlauf durch das Medium erfährt, so sind, dass eine Bedingung (eine physikalische Größe wie Temperatur, Druck, Licht, Strahlung einer bestimmten Intensität oder Wellenlänge etc. oder eine chemische Größe wie pH-Wert, Ionenstärke, Konzentration eines bestimmten Anions oder Kations, ein bestimmtes Löslichkeitsprodukt, die Konzentration einer bestimmten chemischen Spezies, beispielsweise eines bestimmten Moleküls, Ions oder Radikals) einen bestimmten Schwellwert (oder auch Grenzwert oder Threshold) überschreitet oder unterschreitet.

Gemäß der vorliegenden Erfindung erfolgt die abschließende Analyse des Partikels und/oder der Partikelmischung auf die Berichtsfunktion nachdem diese(s) durch das poröse Medium gelaufen ist, d.h. nach dem Durchfluss und/oder Permeation durch das poröse Medium (nachfolgend auch allgemein als "Durchlauf" bezeichnet), und aus dem porösen Medium ausgetreten ist. Dabei ist unter "Austritt" nicht zu verstehen, dass das Partikel von dem Medium in seiner Gänze räumlich getrennt vorliegen muss. Vielmehr ist "nach Austritt aus dem porösen Medium" dahingehend zu verstehen, dass die Analyse des Partikels und/oder der Partikelmischung an einem Ort stattfindet, der von dem verschieden ist, an welchem die physikalischen, chemischen oder biochemischen Parameter des zu untersuchenden Mediums erfasst werden sollen.

Folglich werden erfindungsgemäß Informationen zu den Parametern *in situ* (z.B. im Gestein, in der geologischen Formation oder in einem chemischen Reaktor) von den Partikeln erfasst und nach Durchlaufen dieser Orte *ex situ* analysiert.

Es handelt sich bei dem Partikel daher um ein Analysepartikel (Tracer), bevorzugt um Mikro- und/oder Nanopartikel. Diese haben eine Referenzfunktion (auch Erkennungsfunktion genannt) in Form einer Markierung, die von den erlebten physikalischen, chemischen und biochemischen Parametern unabhängig ist und welche die Erkennung der Partikel nach dem Durchfluss bzw. Permeation durch das Medium ermöglicht. Die Partikel haben zudem auch mindestens eine Berichtsfunktion (d.h. eine weitere Markierungsfunktion, auch Reporting-Funktion oder Story-Telling Funktion genannt), die sich in Abhängigkeit von den erlebten physikalischen, chemischen und biochemischen Parametern irreversibel verändert. Die Berichtsfunktion umfasst eine Erkennung und/oder quantitative und/oder qualitative Erfassung der erlebten bzw. erfahrenen physikalischen, chemischen und biochemischen Parameter während des Durchflusses bzw. der Permeation durch das Medium, bevorzugt durch ein quantitativ und/oder qualitativ analysierbares Signal der zur Berichtsfunktion eingesetzten Stoffe und/oder Flüssigkeiten.

Mit Hilfe des erfindungsgemäßen Verfahrens lassen sich somit Bereiche und Orte untersuchen, die ansonsten nur schwer oder nicht zugänglich sind und sich nicht im Sichtkontakt zum Beobachter befinden, vorzugsweise im Inneren einer geologischen Formation, d.h. nicht auf der Erdoberfläche, oder im Inneren eines Reaktors oder eines menschlichen sowie tierischen Körpers.

Die Berichtsfunktion kann sowohl im Inneren des Partikels enthalten oder an seiner Oberfläche bereitgestellt sein.

Erfindungsgemäß umfasst die Berichtsfunktion mindestens einen Fluoreszenz-Marker oder einen Marker für die plasmonische Eigenschaft. Diese können einzeln oder in Kombination vorliegen. Die Berichtsfunktion kann mit einer semipermeablen oder permeablen Membran als Trennschicht, oder veränderbarer Schicht nach Außen abgeschirmt sein und/oder zur nachfolgenden oder davorliegenden Referenzfunktion.

Die Berichtsfunktion kann vorzugsweise chemische Marker oder Biomarker umfassen, die die Anwesenheit einer bestimmten chemischen oder biologischen Spezies nachweisen.

Alternativ oder in Kombination wird als Berichtsfunktion eine quantitative und/oder qualitative Veränderung am Partikel selbst genutzt. Hierzu erfährt die Berichtsfunktion bzw. das Partikel selbst eine Veränderung durch die Behandlung der umgebenden physikalischen, chemischen oder biochemischen Parameter. Diese Veränderung erfolgt beim Durchlauf durch das poröse Medium. Die Partikel bzw. Partikelmischungen sind nicht im natürlichen Ökosystemsystem vorhanden, sondern werden im Rahmen des erfindungsgemäßen Verfahrens eingebracht.

Wie vorstehend beschrieben, werden die Partikel in ein Fluid eingebracht. Allgemein kann das Fluid, das durch das poröse Medium geleitet wird, nicht nur eine Flüssigkeit sein, sondern auch ein Gas, ein Gel oder ein Flüssigkeits-Gas-Gemisch, eine Emulsion, ein Nebel (Flüssigkeitströpfchen werden mit einem Gas mitgeführt) oder ein Aerosol. Das Fluid kann folglich beispielsweise Wasser, Öl, ein Öl-Wasser-Gemisch, ein erzeugter oder natürlicher Gasstrom, ein Flüssigkeits-Gas-Gemisch, Dämpfe, aber auch Nebel sein.

Die Partikel, insbesondere Nanopartikel, mit der Referenz- und Berichtsfunktion können sich dabei in jeder Komponente solcher Misch-Fluide befinden. Sie können sich in einer Komponente oder in mehr als einer Komponente befinden, beispielsweise sowohl in den Flüssigkeitströpfchen als auch in der Gasphase eines Nebels oder aber nur in einer von beiden.

Das Fluid kann auch Additive und/oder Detergenzien enthalten. Vorzugsweise besteht der Gasstrom aus Luft, Industriegasen oder Edelgasen oder Gemischen daraus.

Die Analyse des Partikels und/oder der Partikelmischung erfolgt vorzugsweise durch optische Spektroskopie, IR-Spektroskopie, plasmonischer Resonanz, Mikroskopie, Dosimetrie, Kernspinresonanz, Elektronenspinresonanz, ENDOR, Fluoreszenzspektroskopie, Einzelmolekülfluoreszenzspektroskopie, Atom-Fluoreszenzspektroskopie, Lumineszenz-Spektroskopie, Photolumineszenz-Spektroskopie, Chromatographie, Gaschromatographie, Flüssigkeitschromatographie und/oder Hochleistungsflüssigkeitschromatographie (HPLC).

Eine Variante des erfindungsgemäßen Verfahrens entnimmt die Partikel oder das Fluid mit den Partikeln und führt noch eine Folgereaktion durch, die den Nachweis der Veränderung der Berichtsfunktion erleichtert. Eine Möglichkeit des Nachweises besteht beispielsweise durch Verwendung von Teststreifen, ähnlich wie in der Medizin und in der Pharmazie üblich, auf denen sich das entsprechende Nachweisreagenz befindet. Dieses kann z.B. seine Farbe verändern, wenn die Berichtsfunktion der Partikel sich beim Durchlauf durch das poröse Medium verändert hat.

Das erfindungsgemäße Verfahren wird verwendet zur Quantifizierung von Gesteinen, Gesteinsschichten und/oder porösen Materialien oder Schichten aus diesem porösen Material, bei der geologischen Untersuchung von Gesteinen, Gesteinsschichten und/oder porösen Materialien oder Schichten aus diesem porösen Material, in der Hydrologie, Gewässererkundung, Lagerstättenerkundung, Lagerstättenüberwachung, Fracking, Geothermie, Dichtigkeitsüberwachung, Überwachung chemischer, biologischer und/oder biotechnologischer Reaktoren, von Wasserbehältern, Wasserspeichern und Wasserleitungssystemen oder in medizinischen In-vivo-Verfahren.

Insbesondere ist das erfindungsgemäße Verfahren geeignet zur hydrologischen und/oder geologischen Untersuchung von Gesteinen, Gesteinsschichten und/oder porösen Materialien oder Schichten aus diesem porösen Material.

Erfindungsgemäß werden hierbei durch direkten oder indirekten Nachweis von physikalischen, chemischen oder biochemischen Parametern am einzelnen erfindungsgemäßen Partikel durch die Berichtsfunktion folgende Parameter erfasst:
Physikalischen Bedingungen, zum Beispiel Druck, Temperatur, Strahlung, oder auch chemische Bedingungen, zum Beispiel der pH-Wert, als ein Maß für den sauren oder basischen Charakter einer wässrigen Lösung, oder die Ionenkonzentrationen einer chemischen Verbindung oder eines Elements.
Die Konzentration, Art und/oder das Vorkommen von Gasen, wie beispielsweise Sauerstoff, Stickstoff, gasförmigen Verbindungen von Elementen des Periodensystems (Periodensystem der Elemente) oder gasförmigen Kohlenwasserstoffen.
Konzentration, Art und/oder das Vorkommen von Elementen des chemischen Periodensystems, deren Salze, Ionen oder deren kovalenten Verbindungen untereinander, beispielsweise Alkalimetalle, Nichtmetalle, Metalle, Halbmetallen, Edelgasen, Lanthanoide oder Actinoide.
Konzentration, Art und/oder das Vorkommen von organischen Makromolekülen oder organisch-anorganischen Makromolekülen, deren Salze oder ionischen Verbindungen, zum Beispiel Fetten oder Ölen.
Konzentration, Art und/oder das Vorkommen von Biomolekülen, zum Beispiel Huminsäuren, Proteine, Nukleinsäuren oder Ribonukleinsäuren.

Die Erfassung der physikalischen, chemischen oder biochemischen Parameter am einzelnen Partikel erfolgt vor Ort im Inneren bei der Durchströmung und/oder Permeation des porösen Mediums.

Unter Permeation oder Diffusion versteht man den Vorgang der Durchdringung, Durchlaufens oder Durchwanderns, bei dem ein Stoff (erfindungsgemäß das Partikel) als Permeat, einen Festkörper als poröses Medium durchdringt, durchläuft oder durchwandert. Die Triebkraft ist ein Konzentrations- oder Druckgradient des Permeates oder die Brownsche Molekularbewegung.

Unter Durchströmung versteht man den Vorgang des Durchlaufens von Hohl- und/oder Zwischenräumen zwischen Festkörpern.

Durch den Einfluss der physikalischen, chemischen oder biochemischen Parameter erhält das Partikel mindestens ein Signal, welches das Partikel mindestens durch eine analytische Berichtsfunktion (d.h. äußere Markierungsfunktion und/oder innere Hohlraumfüllung) aufnimmt und speichert und/oder durch Formänderung des Partikels selbst, sogenannter Memory-Effekt. Hierzu werden die Partikel, sortenrein oder in Mischungen aus verschiedenen Partikeln, mittels Flüssigkeits- oder Gasstrom in das Medium eingebracht (z.B. in Gesteinsritzen, Felsen, geologischen Formationen, Kanäle oder Poren) und die im Partikel-Volumen oder an ihrer Oberfläche bzw. den Partikelschichten durch die erfahrenen physikalischen, chemischen und biochemischen Umgebungsbedingungen verändert.

Erfindungsgemäß kommen Partikel zum Einsatz auf der Millimeterskala (5-1 mm), der Sub-Millimeter-Skala (999 µm bis 100 µm) der Mikrometer-Skala (100 µm bis 1 µm) oder der Nanometerskala (999 nm bis 0,4 nm).

Als durchströmende und/oder permeatierende Flüssigkeit, die als Trägermaterial bzw. Durchströmungs- und/oder Permeatflüssigkeit der erfindungsgemäßen Partikel dient, kann beispielsweise Wasser, Öl, oder Öl-Wasser-Gemische, mit oder ohne Zusatz von Additiven und Detergenzien verwendet werden. Als Trägermaterial der erfindungsgemäßen Partikel kann auch ein erzeugter oder natürlicher Gasstrom beispielsweise aus Luft, Industriegase oder Edelgase verwendet werden. Als Fluid sind auch geeignet Flüssigkeits-Gas-Gemische oder Dämpfe, Nebel (Gasphase plus Kondensat).

Die Durchmesser der verwendeten Partikel liegen bevorzugt im Mikrometer- bis Nanometerbereich, d.h. zwischen 0,5 nm und 100 µm, besonders bevorzugt zwischen 10 µm und 5 nm. Bevorzugt sind Partikel zwischen 50 nm und 5 µm. Zum Einsatz kommen die Partikel je nach geologischer oder hydrologischer analytischer Fragestellung sortenrein oder in Mischungen.

Partikel, die sich leicht und preiswert in großen Mengen und mit einer engen Größenverteilung (Dispersität) herstellen lassen, bestehen aus Oxiden oder aus Polymeren oder aus Wachs. Sie können beispielsweise aus Siliziumoxid oder Polystyrol oder aus Olefinen hergestellt werden. In den Partikeln oder auf ihrer Oberfläche lassen sich Moleküle oder kleinere Nanopartikel mit den gewünschten Referenz- oder Berichtseigenschaften einlagern, anlagern, lösen oder befestigen.

Eine weitere sehr relevante Materialklasse sind Partikel aus Metall, etwa aus Silber oder Gold. Durch Adsorption etwa von Thiolen oder von Dithiolen, aber auch von anderen Molekülen, die die plasmonische Eigenschaft verändern, lassen sich die plasmonischen Eigenschaften metallischer Nanopartikel erheblich verändern und damit nachweisen, beziehungsweise das Signal als Berichtsfunktion verwenden. Auch lassen sich Veränderungen durch chemische Reaktionen von auf der Oberfläche der Metall-Nanopartikel chemisorbierten Thiolen oder Dithiolen über die Veränderung der plasmonischen Eigenschaften - (i) Intensität der plasmonischen Absorption, (ii) Breite der entsprechenden Absorptionsbande und (iii) Lage des Absorptionsmaximums - nachweisen. Die metallischen Nanopartikel kombinieren in sehr vorteilhafter Weise Berichts- und Referenzeigenschaften: auch die "nackten" Nanopartikel zeigen die plasmonische Resonanz. Durch die Adsorbate und Chemisorbate wird diese jedoch verändert: es entwickelt sich die verschobene Resonanz und es nimmt die Resonanz mit dem ursprünglichen Resonanzspektrum ab.

Als Material für die Partikel werden daher vorzugsweise metallische Nanopartikel verwendet, beispielsweise aus Silber, Gold oder Kupfer. Durch Adsorbate, Chemisorbate und die Wechselwirkung mit dem umgebenden Medium wird deren Plasmaresonanz verändert, die sich spektroskopisch messen lässt. Diese Partikel können leicht hergestellt werden und sind kommerziell gut erhältlich.

Als Material für die Partikel werden vorzugsweise auch Polymerpartikel verwendet, insbesondere die sehr leicht herstellbaren und kommerziell erwerblichen Polystyrol-Latexkugeln (Polystyrene Latex Spheres) oder Siliziumoxid-Partikel (Silica Spheres), die ebenfalls leicht mit enger Größenverteilung und in sphärischer Form herstellbar und auch kommerziell erwerblich sind.

Die Partikel können unterschiedliche Form und Gestalt haben. Bevorzugt sind für viele Anwendungen sphärische Partikel (beispielsweise Polystyrol-Latex-Kugeln oder Silica Spheres) oder runde, langgestreckte, abgerundete oder stäbchenförmige Partikel, beispielsweise in Form von Mizellen, ausgeführt als hohle Strukturen, gefüllte Strukturen oder Kern-Schale-Partikel. Daneben kommen auch plättchenförmige Strukturen, z. B. mineralische Scheibchen aus Glimmer oder Laponit in Frage, die sich funktionell beschichten lassen, sowie - im Besonderen im Bereich der Polymere und der Biopolymere - auch unregelmäßig geformte Strukturen. Bei Polymer- und Biopolymer-Partikeln lässt sich insbesondere auch ihre (i) Faltung und (ii) Konformation sowie (iii) ihr Quellungsgrad für den Nachweis nutzen. Viele Polymere neigen in Lösung zur Knäuelung, die sich unter bestimmten Bedingungen wie pH-Wert oder Ionenkonzentration, lockert. Alle drei genannten Parameter hängen stark von den Umgebungsbedingungen ab. Allerdings sind diese Veränderungen meist reversibel. Bei der Erhöhung des Quellungsgrades bzw. der Lockerung der Knäuelung werden eingelagerte Moleküle entweder freigesetzt oder durch den Kontakt mit dem Fluid (z.B. dem umgebenden Wasser) so verändert, dass sie irreversibel freigesetzt und/oder verändert werden und diese Veränderung kann - beispielsweise mit optischer Spektroskopie, Infrarotspektroskopie, Ultraviolettspektroskopie oder Fluoreszenzspektroskopie als Berichtseigenschaft nachgewiesen werden.

Es lassen sich neben (1.) klassischen Kern-Schale-Partikel, bei welchen das Partikel von einer Schale in Form einer Beschichtung umgeben ist, beispielsweise Partikel aus Silica, Umhüllung aus Polystyrol oder einem anderen Polymer oder Umhüllung eines Partikels mit Wachs, Paraffin oder einer Fettschicht, die bei einer bestimmten Temperatur schmilzt, auch (2.) Partikel verwenden, die aus einem Kern und einer molekularen Schicht bestehen, die den Kern - bevorzugt lückenlos - umhüllt. Ein Beispiel hierfür ist die Umhüllung eines metallischen Mikro- oder Nanopartikels, bevorzugt aus Münzmetallen wie Kupfer, Silber, Gold, aber auch von Nanopartikeln aus Metalllegierungen, mit einer Schicht aus Thiolen, Dithiolen und Thiol-Derivaten. Es sind hier aber auch Partikel aus Oxiden von Metallen und Metalllegierungen, aus Silica, Aluminiumoxid, Titandioxid und auch Partikel aus Polymeren, denkbar, die von einer monomolekularen Schicht umgeben sind. (3.) Ferner lassen sich unterschiedliche Arten von hohlen Partikel verwenden, bei denen der innere Hohlraum mit a) mindestens einem Gas, b) dem umgebende Fluid, beispielsweise Wasser, oder c) einem anderen Fluid als dem umgebende Fluid, beispielsweise ein Öl, Fett, Silikonöl und/oder eine Fettsäure ganz oder teilweise gefüllt ist. Bevorzugt haben die vorgenannten Stoffe oder darin enthaltende chemische Stoffe und/oder Partikel eine Markierungsfunktion als Berichts- und/oder Referenzfunktion. Oder d) eine poröse Substanz mit gasgefüllten Hohlräumen oder e) eine poröse Substanz mit Flüssigkeitsgefüllten Hohlräumen.

Ist die Schale starr und zerbrechlich, der Kern aber kompressibel, so zerbricht die Schale unter Druckbelastung und das Innere wird dem umgebenden Fluid (z.B. dem Wasser) ausgesetzt. Geschieht nun zwischen einer wasserempfindlichen Komponente im Inneren des Partikels und dem Wasser eine irreversible chemische Reaktion, so wird diese Komponente mindestens eine physikalische oder chemische Eigenschaft verändern, die sich dann als Berichtseigenschaft im Sinne der vorliegenden Erfindung nutzen lässt. Damit lässt sich der Nachweis erbringen, dass der erforderliche Druck zum Kollabieren des Partikels auf seinem Weg (z.B. durch das Gestein) erreicht wurde. Der Schwellwert-Druck, also der Mindestdruck, der erreicht werden muss, damit das Partikel unter äußerem Druck zerbricht bzw. kollabiert, lässt sich sehr leicht einstellen, und es lassen sich maßgeschneidert Partikel herstellen mit unterschiedlichem Schwellwert-Druck. Der Druck, der mindestens erreicht werden muss, bis das Partikel kollabiert, steigt bei gegebener Substanz für die Schale und gegebener Füllung mit zunehmender Schalendicke und mit abnehmendem Partikeldurchmesser an.

Erfindungsgemäß wird mindestens ein analytisches Partikel (Tracer) mit Referenz- und Berichtsfunktion eingesetzt. Aber auch eine Kombination aus mehreren analytischen Partikeln mit oder ohne poröser Trennschicht sind möglich. Beispielsweise Partikelmischungen, bestehend aus einem gefüllten Partikel und einem Partikel mit einem zentralen Hohlraum und einer umgebenden Hülle, wobei die Markierungsfunktion mit der Berichtsfunktion und/oder der Referenzfunktion jeweils (i) auf die Hülle aufgebracht, (ii) in die Hülle integriert oder (iii) innerhalb des Hohlraums befindlich sein kann.

Möglich ist aber auch die Kombination aus mehreren gleichen oder verschiedenen Markierungsfunktionen (als Berichts- und/oder Referenzfunktion) mit oder ohne permeablen Trennschichten und mit oder ohne einer permeablen Außenhülle, wobei auch die Markierungsfunktion oder ein analytischer Tracer selbst die Außenhülle des Partikels darstellen kann. Der Hohlraum kann wahlweise eine gelartige, pastöse oder gasförmige Füllung enthalten (siehe oben). Diese Füllung kann selber als analytisches Medium dienen oder die physikalischen Eigenschaften des Partikels, wie Gewicht oder Auftriebseigenschaften, wie beispielsweise dem statischen Auftrieb, verändern, beispielweise durch Kollabieren der Hülle nach Erreichen (Überschreiten oder Unterschreiten) eines bestimmten Druckes, pH-Wertes, Temperatur etc. Die drei vorgenannten Materialsysteme sind allerdings nur die Ausgangspartikel für die herzustellenden erfindungsgemäß verwendeten analytischen Partikel mit der Markierungsfunktion, in der Art einer Berichtsfunktion und Referenzfunktion. Die Implementierung der Berichts- und Referenzfunktion bei den jeweiligen Partikeln erfolgt durch Aufbringen einer Adsorbat- oder Chemisorbat-Hülle auf die Oberfläche der Partikel und/oder durch Einbringen der Berichts- und Referenzfunktionen - beispielsweise durch Einbringen geeigneter chemischer Moleküle als Markierungsfunktion - in das Innere der Partikel, etwa während des Partikel-Herstellungsprozesses oder durch anschließendes Eindiffundieren und/oder das Füllen eines Hohlvolumens des Partikels. Auch kann das Partikel in zwei Halbschalen hergestellt werden, diese mit der jeweiligen Markierungsfunktion gefüllt werden und danach zwei Halbschalen schlussfest verbunden werden.

Im Folgenden werden zwei konkrete Ausführungsbeispiele für die Partikel mit der Referenzfunktion und der mindestens einen Berichtsfunktion genauer beschrieben.

### Kern-Schale-Version:

Das Partikel wird von einer Schale aus einem anderen Material oder aus dem gleichen Material in einer anderen Struktur umgeben. Beispielsweise kann der Kern die Referenzfunktion, die Schale die Berichtsfunktion enthalten oder umgekehrt. Besonders interessant ist die Variante, bei der die Berichtsfunktion sich im Kern befindet und auf den Kontakt mit Wasser reagiert. Die Hülle schützt den Kern vor Wasser. Solange also die Hülle existiert und diffusionsdicht für Wasser ist, ändert sich die Berichtsfunktion im wasserempfindlichen Inneren (Kern) nicht. Umgibt man den Kern nun mit einer wasserdiffusionsdichten Schale, die bei Erreichen einer bestimmten Schwellwertbedingung (Erreichen einer bestimmten Temperatur, Überschreiten oder Unterschreiten eines bestimmten pH-Wertes, Ausführen einer bestimmten chemischen oder biochemischen Reaktion) für Wasser durchlässig wird, so lässt sich dies durch die Veränderung der Berichtsfunktion im Kern des Partikels durch den Kontakt mit dem Wasser nachweisen. Dies lässt sich bevorzugt auf vier verschiedene Weisen durchführen:
Erstens: Die Schale wird bei Erreichen der Schwellwertbedingung porös.
Zweitens: Die Schale verliert bei Erreichen der Schwellwertbedingung ihre Diffusionsdichtheit gegenüber Wasser.
Drittens: Die Schale löst sich bei Erreichen der Schwellwertbedingung auf.
Viertens: Die Schale löst sich bei Erreichen der Schwellwertbedingung vom Kern ab.

Ein Beispiel ist das Schmelzen der Schale bei Erreichen einer bestimmten Temperatur oder die Auflösung der Schale bei Erreichen eines bestimmten pH-Wertes.

### Partikel-in-Partikel-Variante:

Neben der Kern-Schale-Version kann die vorliegende Erfindung durch eine Partikel-in-Partikel-Variante implementiert werden. In die größeren Partikel werden kleinere Partikel eingebracht, beispielsweise Metall-Nanopartikel in Polymerpartikel, etwa durch eine Fällungsreaktion. Die Form der Partikel kann beispielsweise rund oder sphärisch ausgeprägt sein. Mit der Formgebung der Partikel kann die Eigenschaften des Partikels für den dynamischen Auftrieb im Fluid bzw. die Durchströmung oder Permeation des porösen Mediums beeinflusst und angepasst werden.

Erfindungsgemäß kann das Partikel neben der Referenz- und Berichtsfunktion noch eine weitere Zusatzfunktion aufweisen. Bei diesen trifunktionellen Partikeln sind ebenfalls Nanopartikel mit den vorstehend genannten Durchmessern bevorzugt. Diese Zusatzfunktion kann eine Timerfunktion sein, die anhand eines zeitabhängigen Zerfalls oder einer zeitabhängigen Veränderung einer Eigenschaft die Zeit des Partikels von der Injektion bis zum Nachweis nach Verlassen des porösen Mediums zu bestimmen erlaubt. Radioaktiver Zerfall ist hier nur ein Beispiel. Chemischer Zerfall, die Umwandlung eines Isomers in ein anderes, stabileres Isomer oder eine Oxidation unter Verlust der fluoreszierenden Eigenschaft sind weitere Beispiele.

Diese Zusatzfunktion kann ferner eine magnetische Funktion sein. Die Verwendung trifunktioneller Nanopartikel, deren dritte Funktion ihre magnetischen Eigenschaften, bevorzugt ihr Ferromagnetismus, ist. Dies kann beispielsweise dadurch realisiert sein, dass die Partikel neben Ihrer Berichtsfunktion und ihrer Referenzfunktion zusätzlich noch magnetische Einschlüsse enthalten. Dies erleichtert später die Konzentration und Entnahme der Partikel aus dem Fluid unter Verwendung von Magneten und magnetischen Feldern. Beispielsweise kann das Fluid nach Verlassen des porösen Mediums durch ein Gitter oder Netz fließen, das magnetische Eigenschaften hat und die Partikel anzieht und festhält.

Metallische Nanopartikel, bevorzugt aus Gold oder Silber, zeigen eine plasmonische Resonanz, die mit optischer Spektroskopie oder IR-Spektroskopie nachgewiesen werden kann. Bei Adsorption bestimmter chemischer Moleküle, bevorzugt Thiole, verschiebt sich diese Resonanz. Das Vorhandensein der metallischen Nanopartikel lässt sich spektroskopisch immer durch die Existenz der plasmonischen Resonanz nachweisen, als Referenz-Funktion.

Die Frage, ob die Partikel auf ihrem Weg durch das poröse Medium Thiolen begegnet sind, lässt sich aus der spektralen Lage der Resonanz nach Passieren des Mediums bestimmen: Bei Adsorption der Thiole verschiebt sich die spektrale Lage der plasmonischen Resonanz. Auch der Anteil der Partikel, die den Thiolen begegnet sind, lässt sich so ermitteln. Das plasmonische Spektrum lässt sich zerlegen in einen Anteil, der nicht verschoben ist, und einen Anteil, der verschoben ist.

Nachfolgend wird die reversible bzw. teilreversible Ausführung der Berichtsfunktion beschrieben, wobei eine irreversible Veränderung der Berichtsfunktion durch die erfahrenen Bedingungen erfindungsgemäß ist. Hat man eine Berichts-Funktion, die sich aufgrund einer bestimmten erfahrenen Bedingung verändert hat und die dann, wenn diese Bedingung nicht mehr vorliegt, sich langsam wieder in den Ausgangszustand zurückverändert, so lässt sich darauf schließen, wie lange die Erfahrung zurückliegt und damit auch indirekt, wo, beispielsweise in einer Gesteinsschicht, die genannten Bedingungen herrschten.

Eine besondere Ausführungsvariante ist die Verwendung eines bifunktionellen Moleküls als Partikel mit Reporting Unit und Reference Unit, nachfolgend auch "Two-in-One"-Lösung genannt. Hier sind die Berichts- und Referenz-Einheit nicht getrennte Einheiten, sondern in einer Einheit vereinigt.

Beispiel: Metallische Nanopartikel, beispielsweise aus Gold oder Silber, zeigen eine plasmonische Resonanz, die mit optischer Spektroskopie oder IR-Spektroskopie nachgewiesen werden kann. Bei Adsorption bestimmter Moleküle (beispielsweise Thiole) verschiebt sich diese Resonanz. Das Vorhandensein der metallischen Nanopartikel lässt sich spektroskopisch immer durch die Existenz der plasmonischen Resonanz nachweisen (Referenz-Funktion). Die Frage, ob die Partikel auf ihrem Weg durch das poröse Medium Thiolen begegnet sind, lässt sich aus der spektralen Lage der Resonanz nach Passieren des Mediums bestimmen: Bei Adsorption der Thiole verschiebt sich die spektrale Lage der plasmonischen Resonanz. Auch der Anteil der Partikel, die den Thiolen begegnet sind, lässt sich so ermitteln: Das plasmonische Spektrum lässt sich zerlegen in einen Anteil, der nicht verschoben ist, und einen Anteil, der verschoben ist. Die reversible bzw. teilreversible Ausführung der Berichts-Funktion: Grundsätzlich ist an die als Ganzes und/oder teilweise irreversible Veränderung der Berichts-Funktion durch die erfahrenen Bedingungen gedacht. Hat man eine Berichts-Funktion, die sich aufgrund einer bestimmten erfahrenen Bedingung verändert hat und die dann, wenn diese Bedingung nicht mehr vorliegt, sich langsam wieder in den Ausgangszustand zurückverändert, so lässt sich darauf schließen, wie lange die Erfahrung zurückliegt und damit auch indirekt, wo (beispielsweise in einer Gesteinsschicht) die genannten Bedingungen herrschten.

In Abweichung vom obigen Punkt wäre es im Ausnahmefall möglich, dass die Partikel bzw. Moleküle bereits natürlich im System vorhanden sind und nicht erst im Rahmen des erfindungsgemäßen Verfahrens eingebracht werden. Ein praktisches Beispiel für pH-abhängige Partikel sind Partikel, die Moleküle oder molekulare Gruppen enthalten, die oberhalb eines bestimmten pH-Wertes (d.h. bei dessen Überschreitung) sich spalten oder eine bestimmte molekulare Gruppe abspalten. Ein weiteres Beispiel für pH-abhängige Partikel sind Partikel, die Moleküle oder molekulare Gruppen als Berichtsfunktion enthalten, die unterhalb eines bestimmten pH-Wertes (d.h. bei dessen Unterschreitung) sich spalten oder eine bestimmte molekulare Gruppe abspalten. Diese Reaktionen laufen praktisch komplett irreversibel ab, da bei erneutem Über- bzw. Unterschreiten des kritischen pH-Wertes die für die Rückreaktion benötigte Gruppe nicht mehr zur Verfügung steht.

Partikel zum Nachweis einer Temperaturüberschreitung sind Partikel mit einem temperaturunabhängigen, bei höheren Temperaturen beständigen Kern und einer bei Überschreiten einer bestimmten Temperatur (Schwellwertbedingung) schmelzenden und sich vom Kern ablösenden Hülle. Das Partikel verfügt hierbei über mehrere Markierungen mit einer charakteristischen geometrischen Form, beispielsweise ein Kegel, die sich bei unterschiedlicher Erweichungstemperatur im durchlaufenden Medium verändern. Immer, wenn eine bestimmte Temperatur erreicht wird, wird eine bestimmte Markierung weich und verändert seine Form. Daraus lässt sich erkennen, welche Temperatur im Medium erreicht wurde und welche noch nicht. Der Kern kann beispielsweise ein Metallpartikel, ein oxidisches Partikel, beispielsweise aus Siliziumdioxid, Aluminiumoxid oder Titandioxid, oder ein Polymerpartikel, beispielsweise Polystyrol, sein. Die Hülle kann aus einer beliebigen, im festen Zustand nicht wasserlöslichen und bei der gewünschten Temperatur schmelzenden Substanz bestehen. Dies können Wachse, Paraffin, langkettige Olefine oder Alkane oder auch Fettsäuren, Aldehyde oder Ester sein. Wird die Schmelztemperatur der jeweiligen Substanz erreicht, aus der die Schale gebildet wird, so wird die Schale in der Strömung abgelöst oder die Moleküle der Schale lösen sich im Wasser oder in dem polaren Fluid.

Erfindungsgemäß können auch verschiedene Partikel kombiniert werden, die für Temperaturüberschreitungen von verschiedenen Temperaturen empfindlich sind. Beispielsweise die Verwendung von 10 verschiedenen Partikeln, deren Hüllen bei 10°C (Grad Celsius), 20°C, 30°C, 40°C, 50°C, 60°C, 70°C, 80°C, 90°C und 100°C schmelzen und die als Referenz jeweils ebenfalls ein unterschiedliches Molekül verwenden. Damit lassen sich detaillierte Rückschlüsse in 10-Grad-Schritten ziehen, welche Temperaturen überschritten wurden und welche nicht und wieviel Prozent der Partikel welche Temperaturüberschreitung auf ihrem Weg durch das poröse Medium erlebt haben und damit in der genannten Ausführungsvariante beispielsweise ihre Hülle verloren haben.

Ein weiteres Ausführungsbeispiel ist, dass 10 verschiedene Partikel mit 10 verschiedenen Schmelzpunkten der Hülle gleichzeitig oder sequentiell eingebracht werden und die Durchströmung und/oder Permeation beobachtet oder zeitabhängig chromatographisch analysiert werden. In diesem Falle ist die Referenzeigenschaft für alle 10 Partikeltypen gleich. In analoger Weise lassen sich natürlich auch die Untersuchungen unterschiedlicher Eigenschaften (etwa pH-Wert-Überschreitung und/oder Unterschreitung, Temperaturüberschreitung und Sauerstoff-Exposition) zeitgleich oder sequentiell analysieren. Ein weiteres Ausführungsbeispiel für Partikel zum Nachweis einer Temperaturüberschreitung in nichtwässrigen schwach polaren oder apolaren Medien, sind Partikel mit einem temperaturunabhängigen, bei höheren Temperaturen beständigen Kern und einer bei Überschreiten einer bestimmten Temperatur (Schwellwertbedingung) schmelzenden und sich vom Kern ablösenden Hülle. Der Kern kann beispielsweise ein Metallpartikel, ein oxidisches Partikel (etwa aus Siliziumdioxid, Aluminiumoxid oder Titandioxid) oder ein Polymerpartikel (beispielsweise Polystyrol) sein, die Hülle kann aus einer beliebigen im festen Zustand nicht öl-löslichen und bei der gewünschten Temperatur schmelzende Substanz bestehen. Dies können polare Moleküle wie mehrwertige Alkohole und/oder langkettige oder kurzkettige Carbonsäuren sein. Wird die Schmelztemperatur der jeweiligen Substanz erreicht, aus der die Schale gebildet wird, so wird die Schale in der Strömung abgelöst oder die Moleküle der Schale lösen sich im Öl oder in dem apolaren Fluid.

Ein praktisches Beispiel für druckabhängige Nanopartikel sind sogenannte "Hollow Spheres", hohle Partikel, in denen sich Gas und keine Flüssigkeit befindet und die unter bestimmtem hydrostatischem Druck kollabieren. Je nach Durchmesser und Wandstärke lassen sich gezielt unterschiedliche Kollabierungsdrücke einstellen. Das Partikel-Volumen kann dabei mit einer festen, gelartigen, pastösen oder gasförmigen Hohlraumfüllung teils oder ganz gefüllt sein.

Im Folgenden wird die Art der Veränderung der Berichtsfunktion genauer erläutert. Diese kann irreversibel (nachfolgend als "Schwellwert-Variante" bezeichnet) oder ähnlich eines Dosimeters erfolgen.

Die Schwellwert-Variante: Wird beispielsweise eine bestimmte Temperatur erreicht, so verändert sich die Berichtsfunktion einer bestimmten Partikelsorte. Wird eine weitere Temperatur erreicht, so verändert sich die Berichtsfunktion einer weiteren Partikelsorte. Es lässt sich also nachweisen, ob ein bestimmter Parameterwert erreicht, über- oder unterschritten wurde.

Die Dosimeter-Variante: Das Ausmaß der Veränderung hängt von der Stärke und der Zeit der Einwirkung ab. Beispielhaft sei die Veränderung der Berichtsfunktion durch radioaktive Strahlung oder durch UV-Licht genannt. Das Ausmaß der Veränderung ist dann etwa abhängig von der Dosis, d.h. proportional zur Strahlungsintensität und zur Einwirkungszeit - was der Erfindung des möglicherweise kleinsten Dosimeters der Welt, des (Mikro- bzw. Nano-)Partikel-Dosimeters, entspricht. Ganz analog ist auch eine chemische Dosimeter-Variante denkbar. Die chemische Dosis ist dann die Konzentration der nachzuweisenden chemischen oder biochemischen Spezies mal die Zeit der Einwirkung. Im einfachsten Falle kann dies die Dosis der Sauerstoffeinwirkung, also die durch Sauerstoff bedingte oxidative Dosis sein. In Erweiterung dieses Verfahrens können natürlich auch unterschiedliche oxidativ wirkende Spezies existieren, die jeweils eine Oxidation der Berichtsfunktion des Partikels bewirken können und es kann die oxidative Gesamtdosis ermittelt werden. In apolaren Medien, etwa in Öl, lassen sich statt der Partikel auch emulgierte Wassertröpfchen oder andere Tröpfchen einer polaren Flüssigkeit verwenden, ebenso auch Mizellen. Die Tröpfchen oder Mizellen können dann in genau der gleichen Weise Moleküle, molekulare Gruppen oder Partikel oder Nanopartikel mit Referenzeigenschaften und mit Berichtseigenschaften enthalten.

Bei der chromatographische Analyse der Daten, werden die Partikel nicht kontinuierlich, sondern zu einem bestimmten Zeitpunkt injiziert und kommen beispielsweise nach unterschiedlichen Verweilzeiten auf unterschiedlichem Weg durch das geklüftete Gestein bzw. das poröse Medium wieder heraus, um an der Austrittsstelle als Funktion der Zeit untersucht zu werden. Unterschiedliche Verweilzeiten entsprechen dabei auch unterschiedlichen Wegen durch das Gestein bzw. das poröse Medium (ggf. mit Verweilzeit nach "Trapping" in einer Pore). Der "Erlebnisbericht" der Partikel nach Austritt aus dem porösen Medium als Funktion der Verweilzeit gibt Aufschluss darüber, wie hoch der prozentuale Anteil der Partikel mit Exposition zu den gefragten Bedingungen (z.B. Temperatur über 80°C) als Funktion der Verweilzeit war.

Gemäß einer bevorzugten Ausführungsform der vorliegenden Erfindung werden Partikel verwendet, deren Berichtsfunktion sich bei Überschreiten oder Unterschreiten des Schwellwertes verändert, wobei ein solcher Schwellwert ein scharfer Wert oder ein enger Wertebereich ist, beispielsweise eine Schmelztemperatur der mit Fluoreszenzfarbstoff angefärbten Wachshülle von +/- 10°C, bevorzugt +/- 5°C, besonders bevorzugt +/- 3°C.

Besonders bevorzugte Ausführungsformen des erfindungsgemäßen Verfahrens verwenden Partikel, die durch folgende Merkmale gekennzeichnet sind:
1. Partikel mit einem Partikelkern und einer inneren sowie einer äußeren Hülle,
   wobei (1.) der Partikelkern die Referenzfunktion enthält, beispielsweise metallische Nanopartikel, die plasmonische Resonanzen zeigen,
   wobei (2.) in der inneren Hülle mindestens eine Komponente eingelagert ist, die die Berichtsfunktion aufweist und wasserlöslich oder öllöslich ist, und
   wobei (3.) diese innere Hülle von einer äußeren Hülle umgeben ist, die diffusionsdicht gegenüber dem umgebenden Fluid ist, so dass die in der inneren Hülle eingelagerte Komponente (die die Berichtsfunktion aufweist) von dem Fluid weder herausgelöst noch chemisch verändert noch anderweitig modifiziert, gequollen etc. werden kann, wobei die äußere Hülle dadurch gekennzeichnet ist, dass sie bei Überschreiten oder Unterschreiten des Schwellwertes des zu erfassenden Parameters entweder diffusionsoffen für das Fluid oder die in der inneren Hülle eingeschlossenen Komponente oder für beide wird oder indem sich die äußere Hülle bei Unterschreiten oder Überschreiten des genannten Schwellwertes auflöst und so die in der inneren Hülle eingelagerten, die Berichtsfunktion aufweisenden Komponente freigibt.
2. Partikel gemäß 1., dadurch gekennzeichnet, dass es sich bei dem Partikelkern um ein Mikro- oder Nanopartikel aus Metall (bevorzugt Silber, Gold, Blei, Kupfer, Eisen, Cobalt, ein Metalloxid oder ein ferromagnetisches Material wie Eisenoxid oder Cobaltoxid) oder um Siliziumdioxid oder um Kohlenstoff oder um Polystyrol handelt.
3. Partikel gemäß 1. oder 2., dadurch gekennzeichnet, dass die äußere Hülle eine schmelzbare Hülle ist, die bei einer bestimmten Temperatur oder in einem bestimmten Temperaturbereich schmilzt oder erweicht oder sich in dem umgebenden Fluid auflöst. Alternativ kann auch eine Hülle verwendet werden, die sich bei Über- oder Unterschreiten eines bestimmten pH-Wertes oder einer bestimmten Ionenkonzentration auflöst, erweicht, quillt oder diffusionsoffen wird.
4. Partikel gemäß 1. bis 3., dadurch gekennzeichnet, dass diese äußere Hülle aus Paraffin oder aus Metall oder aus einem schmelzbaren oder in einem bestimmten Temperaturbereich erweichenden oder quellbaren organischen oder anorganischen Polymer oder Oligomer besteht.
5. Partikel gemäß 1. oder 2., dadurch gekennzeichnet, dass die äußere Hülle eine Hülle aus einer öllöslichen Substanz ist, die sich bei Kontakt mit Öl auflöst.
6. Partikel gemäß 1. oder 2., dadurch gekennzeichnet, dass die äußere Hülle eine Hülle aus einem Polymerbrush ist, das auf die innere Hülle aufgepfropft ist, oder dass die äußere Hülle ein Koordinationspolymer ist.
7. Partikel gemäß 1., 2, oder 6., dadurch gekennzeichnet, dass die äußere Hülle eine Hülle aus einem Polyelektrolyt ist. Dieser kann in einer speziellen Ausführungsform auch auf die innere Hülle aufgepfropft sein. Bei der äußeren Hülle kann es sich auch um ein vernetztes oder photovernetztes Polymer handeln.
8. Partikel gemäß 1. bis 7., dadurch gekennzeichnet, dass im Partikelkern Fluoreszenzfarbstoffe oder Quantum Dots oder magnetische Nanopartikel, eingelagert sind.
9. Partikel gemäß 8., dadurch gekennzeichnet, dass es sich bei mindestens einem der verwendeten Fluoreszenzfarbstoffe um Rhodamin oder Rhodaminderivate oder um GFP (Green Fluorescent Protein) oder Ruthenium-Bipyridin-basierte Komplexe und Verbindungen (z.B. Rubpy) handelt.
10. Partikel gemäß 1. oder 7., dadurch gekennzeichnet, dass die äußere Hülle ein Polymer oder Polyelektrolyt ist, das bzw. der oberhalb eines bestimmten pH-Wertes oder einer bestimmten Temperatur diffusionsoffen wird.
11. Partikel gemäß 1., dadurch gekennzeichnet, dass die äußere Hülle eine semipermeable Membran ist.
12. Partikel gemäß 1. bis 11., dadurch gekennzeichnet, dass das Partikel neben dem Partikelkern noch mehr als zwei Hüllen besitzt.

Erfindungsgemäß ist die Referenzfunktion in Form der Markierung im Partikelkern vorzugsweise eingebettet und wird durch das Fluid nicht herausgelöst. Die in der inneren Hülle eingelagerte mindestens eine Komponente, die die Berichtsfunktion aufweist, kann durch das Fluid bei Kontakt mit diesem herausgelöst werden. Folglich kann sie, wenn ein Fluid, in dem sie löslich ist, in Kontakt mit der inneren Hülle kommt, wieder "ausgelagert" werden.

Nachfolgend wird das Verfahren zur Quantifizierung von Gesteinen, Gesteinsschichten und/oder porösen Materialien oder Schichten aus diesem porösen Material, bei der geologischen Untersuchung von Gesteinen, Gesteinsschichten und/oder porösen Materialien oder Schichten aus diesem porösen Material genauer beschrieben. Als Gestein bezeichnet man eine feste, natürlich auftretende, in der Regel mikroskopisch heterogene Vereinigung von Mineralen, Gesteinsbruchstücken, Gläsern oder Rückständen von Organismen. Das Mischungsverhältnis dieser Bestandteile zueinander ist weitgehend konstant, sodass ein Gestein trotz seiner detaillierten Zusammensetzung bei freiäugiger Betrachtung einheitlich wirkt. Eine nachweisbare Eigenschaftsänderung wird dabei durch bestimmte physikalische, chemische oder biochemische Umgebungsbedingungen des zu untersuchenden Materials hervorgerufen. Trifft das Partikel auf seinem Weg durch das poröse Medium oder Gestein auf solche Umgebungsbedingungen (Beispiel: auf eine Temperatur von mindestens 80°C), so wird das Partikel irreversibel verändert, was nach Austritt aus dem porösen Medium oder Gestein durch die Veränderung seiner Eigenschaften nachweisbar ist. Diese Eigenschaftsänderung des Partikels wird nun nach dem Durchströmen und/oder der Permeation der zu untersuchenden Gesteine, Gesteinsschichten und/oder porösen Materialien oder Schichten aus diesem porösen Material nachgewiesen. Dies erfolgt auf beispielsweise auf folgende Weise. An der Austrittsstelle des Fluids, werden entweder hierzu Proben entnommen und das Fluid mit den Partikeln untersucht, oder es werden hierzu Proben entnommen und die Partikel werden - etwa durch Zentrifugation oder Ultrazentrifugation oder durch Sedimentation - angereichert und anschließend untersucht. Separat oder in Kombination mit der Probennahme können die Eigenschaften in Echtzeit in der vorbeiströmenden Flüssigkeit untersucht werden, beispielsweise durch Glasfenster in der Durchflusszelle eines Spektrometers, durch die das Fluid strömt. Die Änderung der Eigenschaften der Partikel kann ganz unterschiedliche Eigenschaften betreffen: Dies kann insbesondere die Absorption elektromagnetischer Strahlung, wie Licht, Ultraviolettstrahlung oder Infrarotstrahlung oder Mikrowellenstrahlung sein. Es kann sich aber auch um eine Änderung der magnetischen Eigenschaften, etwa von ferromagnetischen oder superparamagnetischen Partikeln und Nanopartikeln handeln, die etwa durch oxidative Veränderung durch Einwirkung von Sauerstoff erfolgen kann. Es kann sich aber auch um eine Änderung der dielektrischen Eigenschaften handeln, die ebenfalls etwa durch oxidative Veränderung durch Einwirkung von Sauerstoff erfolgen kann, aber auch durch andere chemische Veränderung des Partikels oder seiner Oberfläche oder des betreffenden Moleküls.

Je nach Art der verwendeten Partikel können Veränderungen der Partikeleigenschaften auch durch NMR und ESR (Kernspinresonanz bzw. Elektronenspinresonanz) nachgewiesen werden. Ebenfalls können die Eigenschaftsänderungen mittels ENDOR (Electron Nuclear Double Resonance) per Magnetresonanz nachgewiesen werden.

Es kann sich aber auch um eine Veränderung der Fluoreszenzeigenschaften handeln, was auf verschiedene Weise möglich ist: Änderung der Fluoreszenzintensität oder Änderung der Fluoreszenz-Wellenlänge oder Auftreten neuer Fluoreszenzen oder Auftreten der Fluoreszenz bei einer anderen Anregungswellenlänge als vorher. Letzteres ist besonders bevorzug für den nachfolgend beschriebenen Nachweis. Bei einer bestimmten Anregungswellenlänge tritt nach Durchströmen des porösen Mediums eine Fluoreszenz auf, die vor Durchströmen des porösen Mediums nicht existent war, oder nur mit geringerer Intensität. Alternativ, bei einer bestimmten Anregungswellenlänge tritt vor Durchströmen des porösen Mediums eine Fluoreszenz auf, die nach Durchströmen des porösen Mediums nicht mehr existent ist, oder nur mit geringerer Intensität.

Nachdem die Partikel und/oder Moleküle das poröse Medium durchströmt und oder durchdrungen (Permeation) haben, müssen sie auf die Veränderung hinsichtlich der Berichtseigenschaft untersucht werden. Im Durchflussverfahren oder in-situ-Verfahren strömt das Fluid mit den Partikeln und/oder Molekülen an einem Sensor bzw. einer Messvorrichtung vorbei, die sowohl das Berichts- als auch das Referenzsignal detektiert. Dies kann etwa durch das durchströmen einer optischen Durchflusszelle in einem Spektrometer oder Fluoreszenzspektrometer geschehen.

Im Batch-Verfahren, einmalig oder mehrfach Probenentnahme und anschließend Untersuchung. Probennahme und Verbringung von Flüssigkeitsvolumina in eine Messvorrichtung im Sinne einer ex-situ-Messung. Durch Probennahme und einen darauffolgenden Anreicherungsschritt, der darauf zielt, die Partikel und/oder Moleküle in höherer Konzentration zu erhalten mit dem Ziel eines stärkeren Messsignals der Berichts- und der Referenzfunktion, bevor die angereicherte Probe zur Messung in die Messvorrichtung verbracht wird. Als Anreicherungsverfahren bieten sich unter anderem Zentrifugation, Ultrazentrifugation oder die Anreicherung durch Verdampfen eines Teils des Fluids an.

Auch sind chemische Fällungsreaktionen denkbar, bei denen die Partikel oder Moleküle aus dem Fluid ausgefällt werden und anschließend stark angereichert untersucht werden können. Dabei ist die Anwendung der Methode nicht auf die Durchfluss-Anwendungsvariante beschränkt, bei dem das Fluid mit den Partikeln auf der einen Seite injiziert wird und auf der anderen Seite wieder herauskommt. Auch die Methode des Hineinpumpens und des anschließenden Heraussaugens an der gleichen Stelle ist denkbar. Dabei würde man bei der chromatographischen Analyse "Last-In First-Out" erwarten, dass die am Schluss als Letzte injizierten Partikel als erste wieder herauskommen.

Eine andere Variante ist die Querdiffusion: Man analysiert (ggf. zusätzlich) die Diffusion quer zur Stromrichtung und erhält Informationen über Querdiffusionskonstanten, Verwirbelungen etc. sowie Kanäle der Querverbindung und deren Ausmaß. Bei Bohrlochversuchen wird oft die Reichweite als radiale Einflussentfernung vom Bohrloch verwendet.

Bei Pumpversuchen z.B. entspricht die Ausdehnung des Absenkungstrichters der Reich-weite des Pumpversuchs. Wird zur Reichweite die Grundwassermächtigkeit (bei vollkommenen Brunnen) oder unter der Annahme vernachlässigbarer vertikaler Strömungskomponenten, die Bohrtiefe (bei unvollkommenen Brunnen) bzw. das Testintervall (bei Packertests) berücksichtigt, kann das Einflussvolumen angegeben werden.

Die räumliche Repräsentativität, die einzelne Untersuchungsmethoden erreichen können, hängt neben den hydraulischen Parametern von der Untersuchungszeit und der Größe des Untersuchungsbereichs ab. Dabei werden Methoden, die üblicherweise mit kurzen Testzeiten in einem kleinen Untersuchungsbereich gefahren werden gegenüber Langzeitversuchen mit mehreren Beobachtungsstellen um mehrere Größenordnungen kleinere Einflussvolumina haben.

In einer besonders vorteilhaften Variante des Verfahrens, sendet ein Partikel bei seiner Untersuchung nach Austritt aus den Gesteinen, Gesteinsschichten und/oder porösen Materialien oder Schichten aus diesem porösen Material, zwei Signale aus, welche detektiert werden können. Erstens ein Signal, das die Anwesenheit des Partikels signalisiert und das sich durch die Umgebungsbedingungen nicht verändert und zweitens ein Signal, das ebenfalls für das Partikel spezifisch ist. Aber nicht in gleicher Weise von seiner Umgebung oder dem Fluid ausgesandt wird und das sich durch die Umgebungsbedingungen verändert, wenn gewisse Bedingungen während der Durchströmung und/oder Permeation erreicht wurden (Beispiel: eine Temperatur von mindestens 80°C). Das erstere Signal hat die Funktion eines Referenzsignals ("Reference Signal"), das letztere hat die Funktion eines Berichtssignals ("Reporting Signal"), das über die erfahrenen Bedingungen während des Durchlaufs durch das Gestein, die Gesteinsschichten und/oder porösen Materialien oder Schichten aus diesem porösen Material Auskunft gibt in einer Gedächtnisfunktion (Memory-Effekt) und Auskunft über die erfahrenen Eigenschaften und Signale. Der Vorteil der Kombination von Referenz- und Berichtssignal liegt darin, dass es sich so unmittelbar ermitteln lässt, wieviel Prozent der Partikel die besagten Konditionen gesehen haben.

Ferner lässt sich zu jedem Zeitpunkt die absolute Konzentration der Partikel bestimmen, aber auch die relative Konzentration bezogen auf die Konzentration der Partikel (Zahl der Partikel pro Volumen) bezogen auf die Konzentration beim Eintritt in das poröse Medium). Konkret lässt sich die Kombination von Referenz- und Berichtssignal auf sehr unterschiedliche Weisen realisieren.

Ein vorteilhaftes praktisches Verfahren ist die Kombination von zwei Molekülen oder molekularen Gruppen, die beide auf zwei unterschiedlichen Wellenlängen Fluoreszenzlicht aussenden, wenn sie beispielsweise im blauen oder ultravioletten Spektralbereich angeregt werden.

Eines der beiden verschiedenen Moleküle oder molekularen Gruppen ist stabil gegen die möglichen Umgebungsbedingungen im porösen Medium, das andere hingegen wird bei Erreichen bestimmter Umgebungsbedingungen hingegen zerstört oder irreversibel in seinen Fluoreszenzeigenschaften verändert. Kommt dann ein Partikel, das beide Moleküle oder molekularen Gruppen enthält, nach Durchlaufen des porösen Mediums und nach Erreichen der zur Veränderung erforderlichen Bedingungen in die Detektion, so zeigt sich, dass die Fluoreszenz des ersteren Moleküls unverändert ist, die des letzteren aber nicht. So kann auch genau quantitativ bestimmt werden, wieviel Prozent aller Partikel die Schwellwertbedingung für die Veränderung des zweiten Moleküls auf dem Weg durch das poröse Medium erreicht haben.

Eine weitere Ausführungsform besteht darin, zwei unterschiedliche Partikel, eines mit der Referenzeigenschaft und eines mit der Berichtseigenschaft, in einem größeren Partikel einzulagern oder an seine Oberfläche anzulagern (beispielsweise physisorbiert, chemisorbiert, oder mit einem adhäsiven Agens angeklebt).

Dies können optische Eigenschaften sein, aber durchaus auch magnetische Eigenschaften oder Magnetresonanzeigenschaften, die beispielsweise bei einer Nanopartikelsorte unter dem Einfluss von Sauerstoff verschwinden, bei der anderen hingegen nicht. Anaerobe Situationen im Gestein lassen sich so in diesem Anwendungsbeispiel unmittelbar nachweisen. Die Kombination von zwei oder drei dieser Verfahren erlaubt umfassende Rückschlüsse über den prozentualen Anteil der Partikel, die der fraglichen Bedingung ausgesetzt waren, über die durchschnittliche Dosis (Ausmaß bzw. Konzentration bzw. Intensität der Exposition, integriert über die Zeit) und die durchschnittlichen Laufzeiten bzw. Verweilzeiten des Fluids im Medium. Die Partikelmessungen können kontinuierlich im vorbeifließenden Fluidstrom per Fluoreszenzspektroskopie ggf. mit Echtzeit-Auswertung der Spektren erfolgen. Ein Alarm - z.B. bei der Verwendung als Trinkwasser oder zur Warnung vor Leckage - kann dann praktisch in Echtzeit gegeben werden, und man muss sich nicht auf Stichproben beschränken, sondern kann kontinuierlich beobachten. Auch eine Chromatographie ist auf diese Weise möglich. Man kann diese Informationen mit der Verweilzeit im Gestein oder der porösen Substanz oder dem Höhlensystem korrelieren.

Die Auslesung kann durch Fluoreszenz etc. geschehen. Die Partikel können im Vorbeifluss gemessen werden oder durch Filtration angereichert werden. Eine interessante Variante ist die Verwendung magnetischer Partikel, die gezielt durch Magnetfelder an bestimmte Orte geleitet werden können und die zusätzlich den Vorteil der Entnahme und Anreicherung durch starke Magnetfelder ermöglichen, so dass die Partikel nach der Passage zur Analyse wieder mit magnetischer Hilfe eingesammelt werden können.

In einer vorteilhaften Variante des Verfahrens werden die Partikel, die sich in einem Fluid, beispielsweise Flüssigkeit, dispergiert befinden, mittels Ultraschall behandelt, bevor das Fluid mit den Partikeln für den Durchfluss durch das poröse Medium injiziert wird. Auf diese Weise werden Partikel, die sich teilweise zu Aggregaten koaguliert haben, wieder in einzelne Partikel dispergiert.

Wie vorstehend beschrieben kann die Berichtsfunktion eine beliebige Eigenschaft oder Kombination verschiedener Eigenschaften sein, die, bedingt durch die beim Durchfluss durch das poröse Medium erfahrenen Umgebungsbedingungen, verändert wird oder nicht. Eine für den Nachweis sehr geeignete Eigenschaft ist die Veränderung der chemischen oder biochemischen oder spektroskopischen oder sonstigen Eigenschaften durch Abspaltung einer molekularen Gruppe. Handelt es sich dabei einfach um eine Abspaltung von Wasserstoff in Form von H oder H⁺, so ist diese Reaktion allerdings in der Regel reversibel: H⁺-Ionen sind ubiquitär im wässrigen Medium. Handelt es sich allerdings um komplexere Funktionseinheiten, so wird die Abspaltungsreaktion auch dann irreversibel bleiben, wenn die Reaktionsbedingungen sich während des Durchflusses wieder ändern und die Bedingungen für das Stattfinden der Rückreaktion gegeben wären: Die einmal abgespaltene Gruppe ist längst wegdiffundiert oder mit der Strömung an einen anderen Ort verbracht worden und steht für die Rückreaktion nicht mehr zur Verfügung. Spaltet das Partikel also eine solche Gruppe aufgrund von Umgebungsbedingungen einmal ab, so kehrt diese chemische Gruppe oder dieses Molekülfragment oder Ion nie mehr an den Ausgangsort zurück (aus Wahrscheinlichkeitsgründen) und die Rückreaktion findet nie mehr statt, auch wenn die Bedingungen hierfür später durchaus wieder gegeben wären. Wichtig ist, hierfür als abzuspaltende Gruppe oder Spezies eine Gruppe oder Spezies zu verwenden, die in dem Fluid, das durch das poröse Medium strömt oder gepumpt wird, nicht ohnehin ("ubiquitär") verfügbar ist.

Die abzuspaltende Gruppe kann abgespalten werden als Radikal, als Kation, als Anion, als Molekül oder Molekülfragment oder sogar als Partikel.

Die Abspaltung kann etwa in Form einer chemischen Reaktion erfolgen in Form einer molekularen Sollbruchstelle, die durch Lichtadsorption, UV-Bestrahlung, radioaktive Bestrahlung, pH-Wert, oxidativen Einfluss oder Ionenstärke oder durch spezifische oder unspezifische chemische oder biochemische Reaktionen durch anwesende bestimmte Moleküle oder durch Sauerstoff, durch Ozon, durch Oxidationsmittel aufgebrochen wird.

Neben der Abspaltung ist auch die Blockierung oder das Schützen einer bestimmten molekularen funktionellen Gruppe oder Einheit möglich. Ferner kann auch durch eine Additionsreaktion, Komplexierung etc. eine Gruppe so in ihren Eigenschaften verändert werden, dass sich die Berichtseigenschaft verändert.

Eine wichtige Variante ist der Nachweis bestimmter Ionen über Komplexbildung, die häufig mit einer deutlichen Farbänderung einhergeht, sowie andere Farbreaktionen, die die Reaktion und damit das Vorhandensein der die Reaktion auslösenden Spezies (beispielsweise eines Metall-Ions, Metalls oder Metalloxids) damit sehr einfach spektroskopisch nachweisen lässt. Beispiele sind etwa der Eisennachweis mit Thioglykolsäure oder mit Hexacyanoferraten oder mit Thiocyanaten.

Eine ganz andere Möglichkeit ist die Ausfällung von Partikeln, etwa ab einer bestimmten lonenstärke. Man sendet beispielsweise fünf verschiedene Partikel in das poröse Medium, die alle etwa gleich groß sind, aber mit fünf verschiedenen Erkennungsfunktionen markiert sind, und die sich in der Ionenstärke oder dem pH-Wert unterscheiden, bei dem sie im Fluid (z.B. im wässrigen Medium) nicht mehr dispergiert werden können, sondern ausgefällt werden. Wenn nun alle fünf gleichzeitig am gleichen Ort in das poröse Medium injiziert werden, aber am anderen Ende nur noch zwei oder drei der Spezies herauskommen, so lässt sich daraus schließen, dass die anderen ausgefällt wurden, was unmittelbaren Rückschluss über die erlebte Ionenstärke oder den erlebten pH-Wert liefert.

Magnetische Partikel sind für das erfindungsgemäße Verfahren in verschiedener Hinsicht von großem Interesse: (1) Zum einen erlaubt die magnetische Eigenschaft eine einfache Möglichkeit der Anreicherung nach Durchströmung des porösen Mediums. (2) Und darüber hinaus lässt sich die magnetische Eigenschaft alternativ auch als Berichtseigenschaft verwenden: die magnetische Eigenschaft kann durch vielerlei chemische Reaktionen mit dem vor der Reaktion noch magnetischen Partikel zerstört werden.

Ein interessanter Ansatz ist das systematische Entkleben bzw. Ablösen bei Erreichen der Schwellwertbedingung. Ein zentrales Partikel enthält die Eigenschaft, mit der es erkannt wird, in sich selbst. An seine Oberfläche werden weitere Spezies, beispielsweise weitere (z.B. kleinere) Partikel über eine spezifische Wechselwirkung oder einen "Klebstoff" im weitesten Sinne "angeklebt". Diese Partikel enthalten die Berichtseigenschaft. Ein Beispiel ist die Verwendung von Mikropartikeln, auf deren Oberfläche Nanopartikel aufgeklebt sind. Der Kleber kann ein Wachs oder ein Polymer sein, das bei einer bestimmten Temperatur erweicht oder schmilzt, so dass sich die Klebung löst und die kleinen sich vom großen Partikel ablösen. Reichert man nun beispielsweise durch Zentrifugation die großen Partikel nach Durchfluss durch das poröse Medium an, so fehlt ihnen die Berichtseigenschaft. Eine besonders vorteilhafte Gestaltung dieser Variante ist die Verwendung magnetischer Partikel entweder für die aufgeklebten Partikel oder für das zentrale Partikel. Ist beispielsweise das zentrale Partikel ferromagnetisch und die aufgeklebten Partikel sind fluoreszierend, so lassen sich die zentralen Partikel nach Durchfluss durch das Medium magnetisch anreichern. Zeigen sie keine Fluoreszenz mehr, hat sich auf dem Wege durch das poröse Medium die Klebe-Verbindung zwischen zentralem Partikel und aufgeklebten Partikel gelöst: die Erweichungstemperatur der Klebung war offensichtlich überschritten. Entsprechend können auch Klebungen und Haftungskräfte zwischen den Partikeln verwendet werden, die von der Ionenstärke, vom pH-Wert, von den chemischen und biochemischen Bedingungen oder vom Sauerstoffangriff abhängen.

Sowohl für die Abspaltung chemischer Gruppen als auch für die selektive Entklebung bei Erfahrung bestimmter Bedingungen kann man unter anderem die folgenden verschiedenen Varianten einsetzen: (1) Abspaltung durch Licht (Ultraviolett, Infrarot, sichtbares Licht), (2) Abspaltung durch sonstige elektromagnetische Strahlung, z.B. durch Mikrowellen, (3) Abspaltung durch andere Strahlung, insbesondere durch radioaktive Strahlung (mit der Option der Verwendung als kleinstes Dosimeter), (4) durch die Über- oder Unterschreitung eines bestimmten pH-Wertes induzierte Spaltung, (5) Spaltung durch Einwirkung von Wasser (feuchtigkeitsinduzierte Spaltung, "Humidity-induced Cleavage) und (6) Katalytische Spaltung oder photokatalytische Spaltung (z.B. bei gleichzeitiger Anwesenheit von Titandioxid-Partikeln und Licht).

Letztere Variante zeigt auch beispielhaft eine weitere Variante des Verfahrens, nämlich die Möglichkeit, die gleichzeitige Anwesenheit von mehr als einer Eigenschaft zu überprüfen (Eigenschaftskoinzidenzprüfung): die gleichzeitige Anwesenheit von Licht und Photokatalysator, oder in einem anderen Beispiel: die gleichzeitige Anwesenheit von Sauerstoff und einer Mindesttemperatur für die Oxidationsreaktion.

Das erfindungsgemäße Verfahren findet folglich Verwendung bei der Quantifizierung von Gesteinen, Gesteinsschichten und/oder porösen Materialien oder Schichten aus diesem porösen Material, bei der geologischen Untersuchung von Gesteinen, Gesteinsschichten und/oder porösen Materialien oder Schichten aus diesem porösen Material, in der Hydrologie, Gewässererkundung, Lagerstättenerkundung, Lagerstättenüberwachung, Fracking, Geothermie, Dichtigkeitsüberwachung, aber auch bei der Überwachung chemischer, biologischer und/oder biotechnologischer Reaktoren, oder in medizinischen In-vivo-Verfahren.

Technisch dürfte beispielsweise die Verwendung partikulärer Feuchtigkeitssensoren eine wichtige Rolle spielen. Auch der Nachweis von Öl im Gestein (Änderung der Berichtseigenschaft durch die Anwesenheit von (Spuren von) Öl ist technisch hochrelevant). Gleiches gilt für den Nachweis von Schwermetallen. Hier könnte das poröse Medium beispielsweise der Boden in der Umgebung einer Mülldeponie oder im Einzugsbereich eines Trinkwasserspeichers sein.

Eine potentielle Anwendung ist dabei auch die Sicherstellung der Abdichtung bzw. die Leckage-Ortung bei Mülldeponien, Sondermülldeponien und anderen Lagerstätten. Bringt man in das eingelagerte Gut an bestimmten Stellen solche Tracerpartikel ein, so lassen sich beim Fund solcher Partikel z.B. im Grundwasser oder im Deponieablaufwasser die Stellen, wo die Leckage auftrat, genau zuordnen. Dies gilt auch für die Langzeitüberwachung der Leckage aus Autowaschstraßen, radioaktiv oder chemisch belasteten Leitungssystemen oder Zisternen. Die Überwachung kann äußerst kostengünstig und zugleich kontinuierlich erfolgen.

Das Verfahren eignet sich von der zerstörungsfreien Innenerkundung poröser Medien und deren Eigenschaftskontrolle (als Sicherstellung von Qualität und Eigenschaften bei der Produktion) bis zur Exploration von Lagerstätten von Erdöl, Erdgas, Bodenschätzen, Geothermie, Porosität von Gesteinen und bis hin zur kostengünstigen, zerstörungsfreien Erkundung von Gesteinsschichten, etwa im Bereich des Tunnelbaus, um nur einige Beispiele zu nennen.

Die Erforschung der Parameter im Inneren von Gesteinsschichten und Bodenformationen ist jedoch ein zentrales Problem beispielsweise bei: Geothermie, Gewinnung von Bodenschätzen und Erforschung von deren Lagerstätten, Wege des Wassers im Bereich von Mülldeponien, Endlagerstätten von toxischen und radioaktiven Abfällen oder Fracking. Hydraulic Fracturing oder kurz Fracking (von englisch "to fracture" aufbrechen, aufreißen; Deutsch auch hydraulische Frakturierung, hydraulisches Aufbrechen, hydraulische Risserzeugung oder auch hydraulische Stimulation) ist eine Methode zur Erzeugung, Weitung und Stabilisierung von Rissen im Gestein einer Lagerstätte im tiefen Untergrund mit dem Ziel, die Permeabilität (Durchlässigkeit) der Lagerstättengesteine zu erhöhen. Dadurch können darin befindliche Gase oder Flüssigkeiten leichter und beständiger zur Bohrung fließen und gewonnen werden. In einer wichtigen Anwendungsvariante wird ein gasförmiges Fluid verwendet, um eine Gas-Strömung zu erzeugen und die Partikel und/oder die Partikelmischung wird der Gasströmung zugesetzt. Dies ist etwa im Bereich der Erkundung von Erdgasvorkommen und deren Strömung durch das Gestein sehr anwendungsrelevant.

Das erfindungsgemäße Verfahren eignet sich auch besonders zur Anwendung bei chemischen und biologischen/ biotechnologischen Reaktoren sowie auf Wasserbehälter, Wasserspeicher und Wasserleitungssysteme. Bei diesen Anwendungen ist das Innere der chemischen und biologischen/biotechnologischen Reaktoren sowie das Innere der Wasserbehälter, Wasserspeicher und Wasserleitungssysteme als das "porösen Medium" zu verstehen.

Als spezielle Verwendung kann beispielsweise die Überprüfung, ob in chemischen Reaktoren eine Maximaltemperatur überschritten wird, genannt werden. Dies ist beispielsweise bei Polymerisationsreaktionen von großem Interesse, wo bei Überschreiten gewisser Temperaturwerte thermische Zersetzungsprodukte und Kohlenstoff entstehen, was die Farbe des entstehenden Polymers negativ beeinflusst - und ebenso die elektrischen Isolationseigenschaften. Mit dem erfindungsgemäßen Verfahren lässt sich feststellen, ob und in welchem Umfang solche Temperaturüberschreitungen stattfanden. Sowohl die Temperaturwerte, die überschritten wurden, lassen sich mit den Tracer-Partikeln mit dem entsprechenden Schwellwert in der Berichtsfunktion feststellen, als auch das Ausmaß der Überschreitung. Konkret, wieviel Prozent der Partikel nach Verlassen des Reaktors die Überschreitung anhand ihrer Berichtsfunktion erlebt haben.

Die Untersuchung kann auch durch Spektroskopie der Partikel direkt in dem entsprechenden Reaktionsprodukt, das den Reaktor verlässt, erfolgen oder durch nachträgliche Extraktion der Partikel aus dem den Reaktor verlassenden Reaktionsprodukt, beispielsweise durch Ultrazentrifugation oder durch Extraktion ferromagnetischer Partikel mittels Magnetfeld.

Daneben ist eine weitere Möglichkeit, dass das erfindungsgemäße Verfahren in medizinischen In-vivo-Verfahren verwendet wird. Dabei wird das Fluid mit den Partikeln mit der Referenzfunktion und der mindestens einen Berichtsfunktion in einen menschlichen oder tierischen Körper injiziert, um beispielsweise Diagnoseverfahren am Blutkreislauf, Lymphsystem, Harnsystem, Verdauungstrakt, Lunge und Atemwege, Nase und Nebenhöhlen durchzuführen.

## Patentansprüche

1. Verfahren zur Quantifizierung eines porösen Mediums mit mindestens einem Partikel oder einer Mischung aus Partikeln, wobei die Partikel eine Referenzfunktion und mindestens eine Berichtsfunktion zum Erfassen von physikalischen, chemischen oder biochemischen Parametern des porösen Mediums aufweisen, umfassend die folgenden Schritte:
das Einbringen des Partikels und/oder der Partikelmischung in ein Fluid,
das Durchströmen und/oder die Permeation des Fluids mit dem Partikel und/oder der Partikelmischung durch das poröse Medium, wobei sich die mindestens eine Berichtsfunktion der Partikel bei Überschreiten oder Unterschreiten eines Schwellwertes des zu erfassenden Parameters verändert, während die Referenzfunktion der Partikel unverändert bleibt, und
nach Austritt aus dem porösen Medium, mindestens eine nachfolgende Analyse des Partikels und/oder der Partikelmischung auf die physikalisch, chemisch oder biochemisch veränderte Berichtsfunktion und der Referenzfunktion der Partikel, wobei die Referenzfunktion zur Wiedererkennung der Partikel dient,
wobei die mindestens eine Berichtsfunktion in den Partikeln und/oder auf der Partikeloberfläche enthalten ist, umfassend mindestens einen Fluoreszenz-Marker oder einen Marker für die plasmonische Eigenschaft, und
wobei die Veränderung der mindestens einen Berichtsfunktion irreversibel ist, **dadurch gekennzeichnet, dass** die Referenzfunktion der Partikel in Form eines Markers für die plasmonische Eigenschaft vorliegt, wobei der Marker für die plasmonische Eigenschaft metallische Nanopartikel, die plasmonische Resonanzen zeigen, beispielsweise aus Silber, Gold oder Kupfer, einschließt.

2. Verfahren gemäß Anspruch 1, wobei die Partikel einen Durchmesser von 100 µm bis 0,5 nm haben, besonders bevorzugt einen Durchmesser von 10 µm bis 5 nm, oder ganz besonders bevorzugt einen Durchmesser von 5 µm bis 50 nm.

3. Verfahren gemäß Anspruch 1 oder 2, wobei der Grundkörper der Partikel aus Silber, Gold, Kupfer oder anderen Metallen, Siliziumoxid, Polystyrol, Olefinen, Wachs oder einer Mischung daraus besteht.

4. Verfahren gemäß einem der Ansprüche 1 bis 3, wobei die Veränderung der mindestens einen Berichtsfunktion kontinuierlich mit der erfahrenen Dosis der Strahlung (Strahlenbelastung) oder der oxidativen Belastung ansteigt.

5. Verfahren gemäß einem der Ansprüche 1 bis 4, wobei die Partikel ferner eine Zusatzfunktion aufweisen, die anhand eines zeitabhängigen Zerfalls oder einer zeitabhängigen Veränderung einer Eigenschaft die Verweilzeit des Partikels in dem porösen Medium zu bestimmen erlaubt.

6. Verfahren gemäß einem der Ansprüche 1 bis 5, wobei die Partikel ferner eine magnetische Zusatzfunktion aufweisen.

7. Verfahren gemäß einem der Ansprüche 1 bis 6, wobei die Partikel eine weitere Berichtsfunktion aufweisen, die sich bei Überschreiten oder Unterschreiten eines Schwellwertes eines zweiten zu erfassenden Parameters, der von dem ersten Parameter verschieden ist, verändert.

8. Verfahren gemäß einem der Ansprüche 1 bis 7, wobei das poröse Medium ein flüssigkeits- oder gasgefüllter Raum ist.

9. Verfahren gemäß einem der Ansprüche 1 bis 7, wobei das poröse Medium Gesteine, Gesteinsschichten und/oder ein poröses Material oder Schichten aus diesem porösen Material umfasst.

10. Verfahren gemäß einem der Ansprüche 1 bis 9, wobei die Analyse des Partikels und/oder der Partikelmischung durch optische Spektroskopie, IR-Spektroskopie, plasmonischer Resonanz, Mikroskopie, Dosimetrie, Kernspinresonanz, Elektronenspinresonanz, ENDOR, Fluoreszenzspektroskopie, Einzelmolekülfluoreszenzspektroskopie, Atom-Fluoreszenzspektroskopie, Lumineszenz-Spektroskopie, Photolumineszenz-Spektroskopie, Chromatographie, Gaschromatographie, Flüssigkeitschromatographie und/oder Hochleistungsflüssigkeitschromatographie (HPLC) erfolgt, oder anhand einer Folgereaktion erfolgt, die den Nachweis der Veränderung der Berichtsfunktion erleichtert.

11. Verwendung des Verfahrens nach einem der Ansprüche 1 bis 10 zur Quantifizierung von Gesteinen, Gesteinsschichten und/oder porösen Materialien oder Schichten aus diesem porösen Material, bei der geologischen Untersuchung von Gesteinen, Gesteinsschichten und/oder porösen Materialien oder Schichten aus diesem porösen Material, in der Hydrologie, Gewässererkundung, Lagerstättenerkundung, Lagerstättenüberwachung, Fracking, Geothermie, Dichtigkeitsüberwachung, Überwachung chemischer, biologischer und/oder biotechnologischer Reaktoren, von Wasserbehältern, Wasserspeichern und Wasserleitungssystemen oder in medizinischen In-vivo-Verfahren.

## Claims

1. A method for quantifying a porous medium with at least one particle or a mixture of particles, wherein the particles have a reference function and at least one reporting function for recording physical, chemical or biochemical parameters of the porous medium, said method comprising the following steps:
introducing the particle and/or the mixture of particles into a fluid,
letting the fluid with the particle and/or the mixture of particles flow through and/or permeate the porous medium, wherein the at least one reporting function of the particles changes when a threshold value of the parameters to be recorded is exceeded or fallen below, while the reference function of the particles remains unchanged, and
after exiting the porous medium, at least one subsequent analysis of the particle and/or the mixture of particles for a physically, chemically or biochemically changed reporting function and the reference function of the particles, wherein the reference function serves to recognize the particles,
wherein the at least one reporting function is contained in the particles and/or on the surface of the particles, comprising at least one fluorescence marker or a marker for the plasmonic property.
wherein the change in the at least one reporting function is irreversible,
**characterized in that** the reference function of the particles is in the form of a marker for the plasmonic property, wherein the marker for the plasmonic property includes metallic nanoparticles that exhibit plasmonic resonances, for example, those made of silver, gold, or copper.

2. The method according to claim 1, wherein the particles have a diameter of 100 µm to 0.5 nm, more preferably a diameter of 10 µm to 5 nm, or most preferably a diameter of 5 µm to 50 nm.

3. The method according to claim 1 or 2, wherein the core of the particles consists of silver, gold, copper, or other metals, silicon oxide, polystyrene, olefins, wax or a mixture thereof.

4. The method according to any one of claims 1 to 3, wherein the change in the at least one reporting function increases continuously with the radiation dose (radiation exposure) or oxidative stress experienced.

5. The method according to any one of claims 1 to 4, wherein the particles further have an additional function that allows determining the residence time of the particle in the porous medium on the basis of a time-dependent disintegration or a time-dependent change in a property.

6. The method according to any one of claims 1 to 5, wherein the particles further have a magnetic additional function.

7. The method according to any one of claims 1 to 6, wherein the particles have a further reporting function that changes when a threshold value of a second parameter to be recorded, which is different from a first parameter, is exceeded or fallen below the second parameter.

8. The method according to any one of claims 1 to 7, wherein the porous medium is a liquid- or gas-filled space.

9. The method according to any one of claims 1 to 7, wherein the porous medium comprises rocks, rock strata and/or a porous material or layers made of this porous material.

10. The method according to any one of claims 1 to 9, wherein the analysis of the particle and/or the mixture of particles is carried out by optical spectroscopy, IR spectroscopy, plasmonic resonance, microscopy, dosimetry, nuclear magnetic resonance, electron spin resonance, ENDOR, fluorescence spectroscopy, single-molecule fluorescence spectroscopy, atomic fluorescence spectroscopy, luminescence spectroscopy, photoluminescence spectroscopy, chromatography, gas chromatography, liquid chromatography and/or high-performance liquid chromatography (HPLC), or by means of a follow-up reaction which facilitates detection of the change in the at least one reporting function.

11. Use of the method according to any one of claims 1 to 10 for quantifying rocks, rock strata and/or porous materials or layers of this porous material, in the geological analysis of rocks, rock strata and/or porous materials or layers of this porous material, in hydrology, water exploration, deposit exploration, deposit monitoring, fracking, geothermal energy, leakage monitoring, monitoring of chemical, biological and/or biotechnological reactors, of water tanks, water reservoirs and water supply systems, or in medical in-vivo methods.

## Revendications

1. Procédé permettant la quantification d'un milieu poreux comportant au moins une particule ou un mélange de particules, dans lequel les particules présentent une fonction de référence et au moins une fonction de rapport permettant de détecter des paramètres physiques, chimiques ou biochimiques du milieu poreux, comprenant les étapes suivantes :
introduction de la particule et/ou du mélange de particules dans un fluide,
passage et/ou perméation du fluide comportant la particule et/ou le mélange de particules à travers le milieu poreux, dans lequel l'au moins une fonction de rapport des particules est modifiée lorsque le paramètre à détecter dépasse ou n'atteint pas une valeur seuil, tandis que la fonction de référence des particules reste inchangée, et
après la sortie hors du milieu poreux, au moins analyse ultérieure de la particule et/ou du mélange de particules pour déterminer les modifications physiques, chimiques ou biochimiques de la fonction de rapport et de la fonction de référence des particules, dans lequel la fonction de référence sert à la reconnaissance des particules,
dans lequel l'au moins une fonction de rapport est contenue dans les particules et/ou à la surface des particules, comprenant au moins un marqueur fluorescent ou un marqueur pour la propriété plasmonique, et
dans lequel la modification de l'au moins une fonction de référence est irréversible, **caractérisé en ce que** la fonction de référence des particules est présente sous la forme d'un marqueur pour la propriété plasmonique, dans lequel le marqueur pour la propriété plasmonique comporte des nanoparticules métalliques montrant des résonances plasmoniques, par exemple d'argent, d'or ou de cuivre.

2. Procédé selon la revendication 1, dans lequel les particules ont un diamètre allant de 100 µm à 0,5 nm, de manière particulièrement préférée un diamètre allant de 10 µm à 5 nm, ou de manière encore plus particulièrement préférée un diamètre allant de 5 µm à 50 nm.

3. Procédé selon la revendication 1 ou 2, dans lequel le corps de base des particules est constitué d'argent, d'or, de cuivre ou d'autres métaux, d'oxyde de silicium, de polystyrène, d'oléfines, de cire ou d'un mélange de ceux-ci.

4. Procédé selon l'une des revendications 1 à 3, dans lequel la modification de l'au moins une fonction de rapport augmente de manière continue avec la dose de rayonnement reçue (exposition aux rayonnements) ou le stress oxydant.

5. Procédé selon l'une des revendications 1 à 4, dans lequel les particules présentent en outre une fonction supplémentaire permettant de déterminer le temps de séjour de la particule dans le milieu poreux à l'aide d'une dégradation en fonction du temps ou d'une modification en fonction du temps d'une propriété.

6. Procédé selon l'une des revendications 1 à 5, dans lequel les particules présentent en outre une fonction supplémentaire magnétique.

7. Procédé selon l'une des revendications 1 à 6, dans lequel les particules présentent une autre fonction de rapport qui est modifiée lorsqu'une valeur seuil d'un second paramètre à détecter, lequel est différent du premier paramètre, est dépassée ou n'est pas atteinte.

8. Procédé selon l'une des revendications 1 à 7, dans lequel le milieu poreux est un espace rempli de liquide ou de gaz.

9. Procédé selon l'une des revendications 1 à 7, dans lequel le milieu poreux comprend des roches, des couches de roches et/ou un matériau poreux ou des couches dudit matériau poreux.

10. Procédé selon l'une des revendications 1 à 9, dans lequel l'analyse de la particule et/ou du mélange de particules est effectuée par spectroscopie optique, spectroscopie infrarouge, résonance plasmonique, microscopie, dosimétrie, résonance magnétique nucléaire, résonance paramagnétique électronique, ENDOR, spectroscopie de fluorescence, spectroscopie de fluorescence monomoléculaire, spectroscopie de fluorescence atomique, spectroscopie de luminescence, spectroscopie de photoluminescence, chromatographie, chromatographie en phase gazeuse, chromatographie en phase liquide et/ou chromatographie en phase liquide à haute performance (HPLC), ou à l'aide d'une réaction consécutive facilitant la détection de la modification de la fonction de rapport.

11. Utilisation du procédé selon l'une des revendications 1 à 10 pour la quantification de roches, de couches de roches et/ou de matériaux poreux ou de couches constituées dudit matériau poreux, dans le cadre de l'étude géologique de roches, de couches de roches et/ou de matériaux poreux ou de couches constituées dudit matériau poreux, en hydrologie, dans l'étude des cours d'eau, la prospection de gisements, la surveillance de gisements, la fracturation hydraulique, la géothermie, la surveillance de l'étanchéité, la surveillance de réacteurs chimiques, biologiques et/ou biotechnologiques, de réservoirs d'eau, de citernes et de systèmes de canalisations d'eau, ou dans des procédures médicales *in vivo.*
